# EUROPEAN PATENT APPLICATION

(11) **EP 3 839 508 A1**
(43) Date of publication of application: **23.06.2021**
(21) Application number: 19216592.6
(22) Date of filing: 16.12.2019
(51) Int. Cl.: G01N 33/543

(54) **A METHOD OF DETECTING AND/OR QUANTITATING AN ANALYTE OF INTEREST IN A PLURALITY OF BIOLOGICAL LIQUID SAMPLES**

(71) Applicant: Blink AG, 07745 Jena (DE)
(72) Inventor: STEINMETZER, Katrin, 07743 Jena (DE); HUBOLD, Stephan, 07745 Jena (DE); ELLINGER, Thomas, 07745 Jena (DE); LEMUTH, Oliver, 07743 Jena (DE); ERMANTRAUT, Eugen, 07749 Jena (DE)
(74) Representative: Engelhard, Markus

(57) **Abstract**

The present invention relates to a method of detecting and/or quantitating an analyte of interest in a plurality of biological liquid samples. Furthermore, the present invention relates to a kit and a cartridge for performing a method for detecting and/or quantitating an analyte of interest in a plurality of samples.

SEQUENCE LISTING

<110> Blink AG

<120> A method of detecting and/or quantitating an analyte of interest in a
plurality of biological liquid samples

<130> B33074EP

<160> 6

<170> BiSSAP 1.3.2

<210> 1
<211> 20
<212> DNA
<213> Artificial Sequence

<223> "HCV-Brun sense primer"

<220>
<223> HCV-Brun sense primer

<400> 1
gtggtctgcg gaaccggtga                                                    20


<210> 2
<211> 22
<212> DNA
<213> Artificial Sequence

<223> "HCV-Brun antisense primer"

<220>
<223> HCV-Brun antisense primer

<400> 2
cgcaagcacc ctatcaggca gt                                                 22


<210> 3
<211> 25
<212> DNA
<213> Artificial Sequence

<223> "HCV-Brun TaqMan probe "

<220>
<223> HCV-Brun TaqMan probe

<400> 3
ccgagtagyg ttgggtygcg aaagg                                              25


<210> 4
<211> 22
<212> DNA
<213> Artificial Sequence

<223> "MS2-Nino sense Primer"

<220>

<223> MS2-Nino sense Primer

<400> 4
ctctgagagc ggctctattg gt                                                22

<210> 5
<211> 24
<212> DNA
<213> Artificial Sequence

<223> "MS2-Nino sense Primer "

<220>
<223> MS2-Nino sense Primer

<400> 5
ggtccctaca acgagcctaa attc                                              24

<210> 6
<211> 25
<212> DNA
<213> Artificial Sequence

<223> "MS2-Nino TaqMan probe "

<220>
<223> MS2-Nino TaqMan probe

<400> 6
tcagacacgc ggtccgctat aacga                                             25

## Description

The present invention relates to a method of detecting and/or quantitating an analyte of interest in a plurality of biological liquid samples.

### BACKGROUND OF THE INVENTION

In the field of diagnostics, sample pooling and sample bar coding strategies have been established and are commonly used in the processing of a large number of biological samples.

Sample pooling strategies in the diagnostic field are typically based on testing a combined pool of defined volumes of different samples. Typically, samples are obtained in duplicates, and one duplicate is kept for storage, whereas the other duplicate is added to the combined pool. If such combined pool is tested positive, then the stored duplicate samples from the same pool are tested individually in order to finally identify positive samples. The process of testing individual duplicate samples from a combined pool that provided a positive result is sometimes also referred to as "re-flexing". In particular, in samples, that are generally expected to provide a negative result for the respective tested analyte, such pooling strategies provide a low-cost approach. However, the extent of pooling has a direct effect on the sensitivity of the test, and re-flexing requires obtaining at least duplicate samples from a single source and careful and controlled storage of such duplicate samples as well as complex test logistics.

Sample barcoding, also sometimes referred to as sample indexing, is a frequently used approach to labeling samples for multiplex sequencing and multiplex analysis. All nucleic acids in a sample are labeled with the same sequence tag, and the resulting library is pooled with other libraries and sequenced in parallel in a single run. Thereafter, the sample-specific indices enable the software to separate the multiplexed sequence data in sample-specific data sets.

Molecular barcoding differs from sample indexing in that each molecule in a sample is labeled with a different and unique sequence prior to PCR amplification. With each nucleic acid in the starting material (i. e. within the sample) being labeled with a unique sequence, i. e. a "molecular barcode" ("MBC"), sequence analysis software can filter out duplicate reads and PCR errors to report unique reads. Sample barcoding as well as molecular barcoding have in common that nucleic acids are tagged with specific molecular sequence labels.

A different kind of barcoding technique is fluorescent cell barcoding (FCB), a cell-based multiplexing technique for high-throughput flow cytometry. Barcoded samples can be stained and acquired collectively, minimizing staining variability and antibody consumption, and decreasing required sample volumes (Krutzik et al., 2011 "Fluorescent cell barcoding for multiplex flowcytometry", Curr. Protoc. Cytom., Chapter 6, Unit 6.31).

In particular in the context of cartridge-based testing, there are limits in terms of the throughput, due to the size and the costs of the cartridge, especially in the context of a one-cartridge/one-sample-approach. Examples of such kind of cartridges are the GenExpert from Cepheid, Idylla from Biocartis, the m-Pima from Abbott and others. A higher throughput is typically achieved by addition of identical instrument resources and running many cartridges at a time. There also exist cartridges that allow for processing multiple samples on a single platform. However, such cartridges are typically designed for performing separate analysis processes for each individual sample, for example for testing a sample for different analytes, and therefore, such cartridges are merely compositions of components that resemble individual analysis tools for the respective sample. Such set-ups are also useful when multiple samples need to be processed in parallel, e. g. a multiplicity of samples are tested for the same analyte, and such set-ups help to increase the throughput on the respective instruments or simply to provide for less hands-on-time with loading samples on the analysis cartridge. One example of such a tool is the BDMAx System. A comprehensive review on existing cartridge based diagnostic tools is given in Relich et al. 2018; "Syndromic and Point-of-Care Molecular Testing", Advances in Molecular Pathology 1(1):97-113.

There continues to be a need in the art to achieve higher throughput and instrument utilization whilst at the same time producing less waste and being less costly . There also continues to be a need for processing multiple samples within one system in a single process that allows for a maximum utilization of hardware and reagent resources. There is furthermore a need in the art to provide for a process that allows to assign the individual sample to be analyzed an individual identity which facilitates performing a single detection reaction on a multiplicity of such individually assigned and identifiable samples.

### SUMMARY OF THE INVENTION

The present invention addresses these and related needs. In one aspect, the present invention relates to a method of detecting and/or quantitating an analyte of interest in a plurality of biological liquid samples, said method comprising two sub-processes, one sample-specific subprocess and one generic subprocess.

The "sample-specific subprocess", herein also sometimes referred to as "sample-specific process part", "specific process part" or "specific part", is a part in which each of the samples is separately subjected to a procedure or conditions in which it is specifically labelled (herein also sometimes referred to as "encoded") such that it may later be identified and distinguished from other samples.

The "generic subprocess", or "generic process part" or "generic part", is a part in which all samples undergo the same manipulations that allow the detection of an analyte. Such generic part does not comprise any sample-specific procedure, handling or manipulation and typically is performed with all the samples under investigation, irrespective of the sample identity.

In one embodiment, the sample-specific process part comprises the following steps:
- separately providing, in any order, a plurality of different biological liquid samples suspected of containing an analyte of interest, and a plurality of differently labelled subsets of porous microparticles, wherein, in said plurality of subsets of porous microparticles, each of said subsets is separate from the other subsets;
- specifically labelling each of said different biological samples by separately exposing each of said separate subsets of microparticles to one biological liquid sample each, thus allowing each sample to be absorbed by one specifically labelled subset of porous microparticles in an aqueous environment;
- separately transferring each subset of porous microparticles from said aqueous environment to a non-aqueous environment;

In one embodiment, said generic process part comprises the steps:
- mixing together the differently labelled subsets of porous microparticles in said non-aqueous environment and thus generating a suspension of a plurality of differently labelled subsets of porous microparticles therein;
- performing a detection reaction for detecting said analyte of interest, on said suspension of said plurality of differently labelled subsets of porous microparticles; and
- detecting and/or quantitating said analyte of interest, if present, in any of said differently labelled subsets of suspended microparticles.

The phrase "in said plurality of subsets, each of said subsets of porous microparticles is separate from the other subsets", and the term "separate subset", as used herein is/are meant to refer to a scenario in which such subset of microparticles is physically separated from other subsets. In one embodiment, two subsets that are "separate" from each other are in different locations and/or may be macroscopically distinguishable and/or are separated by a physical barrier, preferably such that no mixing or cross-contamination between samples can occur. Hence, it is preferred that each "separate subset" is provided in a form that allows the separate handling of such subset. A physical separation of the different subsets may, e.g., be achieved by a barrier surrounding such subset. In a simple embodiment, such separate subset may be located in its own container or compartment. Typically, in accordance with embodiments of the present invention, before a detection and/or quantitation reaction (of an analyte) is performed, the subsets are separate from each other. During use, e.g. during a detection reaction, more specifically during the generic part of such detection reaction, some or all subsets of a library of microparticles may eventually become combined. The term "a plurality of differently labelled subsets of porous microparticles", as used herein, is also sometimes referred to as a "library of microparticles".

The term "biological liquid sample", as used herein, refers to a liquid sample obtained from the body of an organism which may or may not have been further processed, e.g. to make analyte(s) of interest accessible or amenable to further analysis. Preferably such "biological liquid sample" is selected from blood, plasma, serum, urine, sweat, tears, sputum, lymph, semen, ascites, amniotic fluid, bile, breast milk, synovial fluid, peritoneal fluid, pericardial fluid, cerebrospinal fluid, chyle, and urine, wherein, more preferably, said biological liquid sample is plasma. Preferably such biological liquid sample is further processed, e.g. it may be lysed or digested or otherwise treated, before it is subjected to the method according to the present invention. As an example, if the biological liquid sample is plasma, such plasma may have been further lysed and/or digested to get rid of components that may otherwise interfere with the subsequent detection reaction. For example if the analyte of interest is a particular nucleic acid, the biological liquid sample, e.g. the plasma, may be first digested with a protease and other suitable enzymes to remove any unwanted proteins or peptides, as well as lipids or other unwanted components, before it is subjected to the method according to the present invention. A "biological liquid sample", as used herein, may therefore also refer to an extract obtained from any of the aforementioned bodily fluids; it may for example be a nucleic acid extract that has been obtained from any of the aforementioned bodily fluids by way of a suitable extraction, e.g. using cell disruption (if necessary), removal of lipids, proteins and unwanted nucleic acid (if necessary), and purification of nucleic acids and/or enrichment of particular nucleic acids. In some embodiments, a nucleic acid extract may have been produced using ethanol or another suitable alcohol.

As used herein, the term "library" (of microparticles) is meant to refer to a plurality or collection of microparticles, wherein, in such plurality or collection or library of microparticles, there are at least two different types of such microparticles, herein also sometimes referred to as "subsets". In a preferred embodiment, there are at least two subsets of prefabricated precursor-microparticles, preferably three or more subsets of prefabricated precursor-microparticles, with each subset having its distinct label component attached to, contained within or otherwise associated with said microparticles within said subset, such that said at least two or more subsets of microparticles differ by the respective label component attached to, contained within or otherwise associated with each subset. In such a library of different subsets, the respective subsets are typically and preferably stored separately from each other, e. g. in different containers. This means that such term "separate subset", as used herein, is meant to refer to a collection or subset of microparticles that is physically separated from other subsets.

### DETAILED DESCRIPTION OF THE INVENTION

In one aspect, the present invention relates to a method of detecting and/or quantitating an analyte of interest in a plurality of biological liquid samples, said method comprising a sample-specific process part, followed by a generic process part; said sample-specific process part comprising the following steps:
- separately providing, in any order, a plurality of different biological liquid samples suspected of containing an analyte of interest, and a plurality of differently labelled subsets of porous microparticles, wherein, in said plurality of subsets, each of said subsets is separate from the other subsets;
- specifically labelling each of said different biological samples by separately exposing each of said separate subsets of microparticles to one biological liquid sample each, thus allowing each sample to be taken up by one specifically labelled subset of porous microparticles in an aqueous environment;
- separately transferring each subset of porous microparticles from said aqueous environment to a non-aqueous environment;
said generic process part comprising the steps:
- mixing together the differently labelled subsets of porous microparticles in said non-aqueous environment and thus generating a suspension of a plurality of differently labelled subsets of porous microparticles therein;
- performing a detection reaction for detecting said analyte of interest, on said suspension of said plurality of differently labelled subsets of porous microparticles; and
detecting and/or quantitating said analyte of interest, if present, in any of said differently labelled subsets of suspended microparticles.

Preferably, in said step of separately exposing each of said separate subsets of microparticles to one biological liquid sample each, each of said separate subsets of microparticles is exposed to, in any order, one biological sample each and a detection composition for performing a chemical or biochemical detection reaction, such that each subset of microparticles takes up the respective biological sample and the detection composition to which it has been exposed.

In one aspect the present invention also relates to a method of detecting and/or quantitating an analyte of interest in a plurality of biological liquid samples, in particular according to the method as outlined in the previous paragraphs, wherein the sample-specific process entails
a. a step of separately providing, in any order, a plurality of separate biological liquid samples suspected of containing an analyte of interest, and a plurality of porous microparticles; each of said porous microparticles having a porous matrix and being configured to receive a volume of liquid in said porous matrix and, preferably, to take up analyte, if present; wherein, in said plurality of porous microparticles, there are different subsets of microparticles provided, each subset of microparticles being characterized by a specific label component that is attached to, contained in or otherwise associated with the respective subset; wherein in said step of providing, the number of different subsets of microparticles provided is at least as big as the number of separate biological liquid samples provided, and wherein furthermore in said step of providing, the different subsets of microparticles are provided separate from each other; wherein, optionally, said different separate subsets of microparticles contain in their respective porous matrix a detection composition comprising reagents for performing a chemical or biochemical detection reaction of an analyte;
b. a step of exposing each separate subset of microparticles to exactly one separate biological liquid sample, thereby allowing each separate subset of microparticles to incubate with a volume of exactly one separate biological liquid sample and to take up such sample or a portion thereof, and, optionally, to accumulate analyte, if present in said sample, in or on the matrix of the microparticle(s); and, further optionally, in case that said different separate subsets of microparticles, when provided in step a), do not yet comprise reagents for performing a chemical or biochemical detection reaction of an analyte, a step of exposing each separate subset of microparticles to a detection composition comprising reagents for performing a chemical or biochemical detection reaction of an analyte thereby allowing each separate subset of microparticles to receive said reagents;
c. transferring each subset of microparticles separately into a non-aqueous phase and removing some or all of the aqueous phase surrounding the individual prefabricated microparticle(s) of said subset(s), thereby creating a plurality of separate subsets of insulated reaction spaces for detecting said analyte, which reaction spaces comprise an aqueous phase including sample and said reagents for performing a chemical or biochemical detection reaction of an analyte, and which reaction spaces are confined to said void volume(s) of said microparticles;

In one embodiment, the generic process comprises
d. mixing the separate different subsets of microparticles in said non-aqueous phase, such that all of said different subsets of microparticles form a suspension of different microparticles in said non-aqueous phase; subjecting said mixed different subsets of microparticles to conditions required for performing a chemical or biochemical detection reaction of an analyte; performing such detection reaction of said analyte of interest; and detecting and/or quantitating said analyte of interest, if present in any of said different subsets of microparticles, by means of a signal generated in said detection reaction in a respective subset of microparticles, if said analyte of interest is present in any member of said respective subset.

In one embodiment, said method further comprises the step
e) determining which sample(s) of said plurality of samples provided in step a) does contain said analyte of interest by determining the identity of the subset(s) of microparticles in which said analyte of interest is detected in step d).

In one embodiment, the identity of the subset(s) of microparticles in step e) is determined by means of the specific label component that is attached to, contained in or otherwise associated with the respective subset of microparticle(s).

In one embodiment, step b) further comprises a substep of generating first record of correlation indicating which separate subset of microparticles is or has been exposed to which sample, and step d) comprises a substep of generating a second record of correlation indicating in which subset of microparticles a signal has been generated in said detection reaction.

In one embodiment, step e) is performed by reference to said first and second records of correlation and by linking said records, thus allowing to determine which sample(s) of said plurality of samples provided in step a) does(do) contain said analyte of interest.

In one embodiment, each of said porous microparticles has a porous matrix which allows to accumulate analyte of interest by
(i) a polymer or polymer mixture that forms said porous polymer matrix or is said polymer matrix; or
(ii) at least one ionisable group, or a plurality of ionisable groups, immobilized on said porous polymer matrix, said ionisable group(s) being capable of changing its(their) charge(s) according to ambient conditions surrounding said precursor-microparticle; or
(iii) at least one charged group, or a plurality of charged groups immobilized on said porous polymer matrix; or
(iv) a combination of any of (i) - (iii);

In one embodiment, each of said porous microparticles has a porous matrix and comprises an analyte-specific reagent (ASR) that is attached to said porous matrix, such analyte-specific reagent allowing an enrichment of an analyte of interest and/or allowing a specific signal or target amplification reaction involving said analyte; wherein said analyte-specific reagent is capable of specifically binding to an analyte of interest.

As used herein, the term "analyte-specific reagent" ("ASR") is meant to refer to a reagent that is capable of specifically targeting or recognizing an analyte of interest. Such specific targeting or such specific recognition manifests itself in the capability of such analyte-specific reagent to specifically bind to such analyte of interest or to specifically react therewith. In one embodiment, an analyte-specific reagent is selected from nucleic acids, including aptamers, spiegelmers, nucleic acid oligomers and nucleic acid primers; antibodies or antibody fragments; non-antibody proteins capable of specifically binding an analyte or analyte complex, and affinity proteins. In a preferred embodiment, an analyte-specific reagent is selected from nucleic acids, in particular nucleic acid oligomers and nucleic acid primers. Nucleic acid primers are particularly suitable for performing a nucleic acid amplification. In one embodiment, when the analyte-specific reagent is a nucleic acid primer, such analyte-specific reagent is, in fact, a pair of primers which flank the region within an analyte of interest that will subsequently be amplified (and detected). Optionally, in such an embodiment (of a pair of primers being an analyte-specific reagent), such analyte-specific reagent may additionally comprise a detectable probe provided together with the primer(s) and allowing the detection of the respective primer(s) and the resultant amplification product(s).

In another embodiment, each of said porous microparticles has a porous matrix but does not comprise an analyte-specific reagent (ASR) as defined in the preceding paragraph. In this embodiment, an enrichment or accumulation of analyte in the microparticles is achieved by the porous matrix alone.

The term "signal or target amplification reaction" as used herein refers to a chemical or biochemical detection reaction, in which either the signal used for the detection of the analyte is amplified, or the target (i.e. analyte) to be detected and/or quantitated, is first amplified and subsequently detected. Typical examples of a target amplification reaction are nucleic acid amplification reactions, such as PCR. Typical examples of signal amplification reactions are immunochemistry reactions, such as immunoassays.

In one embodiment, the analyte-specific reagent is selected from nucleic acids, including aptamers, Spiegelmers; antibodies or antibody fragments; non-antibody proteins capable of specifically binding an analyte or analyte complex, such as receptors, receptor fragments, and affinity proteins; wherein, preferably, said analyte-specific reagent is selected from nucleic acids, in particular nucleic acid oligomers and nucleic acid primers.

In one embodiment, each of said porous microparticles has the same analyte-specific reagent attached to its porous matrix. In one such embodiment, such same analyte-specific reagent is the only analyte-specific reagent that is attached to the microparticles.

The term "the same analyte-specific reagent", as used herein, is meant to refer to a scenario in which there are various analyte-specific reagent molecules all of which have the same specificity for a single particular analyte. As an example, if the analyte-specific reagent is an antibody that is specific for a particular analyte, all analyte-specific reagents that are "the same" with such analyte-specific reagent, have the same specifity for such analyte. Typically, and in many cases, this means that such "same" analyte-specific reagents are identical in terms of their structure and/or sequence, or they differ in structure and sequence only to such an extent that their specificity for the same single particular analyte remains unaffected by these variations.

In one embodiment, in said plurality (or library) of porous microparticles, there are different subsets of microparticles,
with each subset
- having its distinct label component attached to, contained in or otherwise associated with said microparticles of said subset; and all of said different subsets having
- the same analyte-specific reagent attached to or contained in said microparticles of said subsets, said analyte-specific reagent being specific for one analyte of interest;
such that said different subsets of microparticles are identical in terms of the analyte-specific reagent attached or contained, but differ by
- the respective label component attached to, contained in or otherwise associated with said microparticles of each subset;
with each subset being unambiguously defined and identifiable by said respective label component.

In one embodiment in which all of said different subsets have the same analyte-specific reagent attached to said microparticles of said subsets, and in which said analyte-specific reagent is specific for one analyte of interest, said method is a method of detecting and/or quantitating one analyte of interest in a plurality of biological liquid samples, wherein the number of different subsets of microparticles provided, preferably provided in step a), equals the number of separate biological liquid samples provided.

In another embodiment, in said plurality (or library) of porous microparticles, there are several different analyte-specific reagents attached to or contained in said microparticles.

In such embodiment (i.e. wherein, in said plurality of porous microparticles, there are several different analyte-specific reagents attached to or contained in said microparticles), there are different subsets of microparticles,
with each subset
- having its distinct label component attached to, contained in or otherwise associated with said microparticles of said subset; and
wherein furthermore, in said plurality of porous microparticles, there are different classes of subsets of microparticles with each class of subsets
- having a different analyte-specific reagent attached to the porous matrix of said microparticles or contained in said microparticles; wherein, preferably, there are at least two different classes of subsets of microparticles, more preferably at least three or more different classes of subsets of microparticles;
such that said different subsets of microparticles differ by the respective label component attached to, contained in or otherwise associated with said microparticles of each subset; and each subset of microparticles forms part of one class of subsets of microparticles; with each subset being unambiguously defined and identifiable by the respective label component and the respective analyte-specific reagent; and
such that said different classes of subsets of microparticles differ by the respective analyte-specific reagent attached to the porous matrix of said microparticles; and each of said different classes comprises several subsets of microparticles, all of which have the same analyte-specific reagent attached or contained.

In such embodiment (i.e. wherein, in said plurality of porous microparticles, there are several different analyte-specific reagents attached to or contained in said microparticles), said method is a method of detecting and/or quantitating more than one analyte of interest in a plurality of biological liquid samples, wherein, the number of different subsets of microparticles provided, preferably provided in step a), equals the number of separate biological liquid samples provided, multiplied by the number of analytes of interest to be detected, and wherein there are as many classes of subsets of microparticles provided, preferably provided in step a), as the number of analytes of interest to be detected.

It should be noted that there are numerous types of microparticles that may be used in accordance with the present invention, as long as they have a porous matrix, are configured to receive a volume of liquid in said porous matrix and have a specific label component attached to, contained in or otherwise associated with the microparticle and which label component allows to classify such microparticle to belong to a particular subset of microparticles. Suitable microparticles may or may not have analyte-specific reagents (ASRs). It has turned out that microparticles, such as are disclosed in the parallel EP application entitled "A library of prefabricated microparticles and precursors thereof' (attorney docket B33016EP) by the present applicant Blink AG, are particularly suitable also in embodiments of the present invention, in particular in such embodiments where more than one analyte is to be detected.

In one embodiment, said porous matrix is a porous polymer matrix formed by a polymer or polymer mixture, wherein, preferably, said porous polymer matrix is composed of a polymer (or polymers) that is(are) not crosslinked, wherein, more preferably, said polymer or polymer mixture that forms said porous polymeric matrix, is composed of agarose or a combination of agarose and gelatin, wherein, more preferably, in said combination of agarose and gelatin, said agarose is present in a range of from 0.1 %(w/v) to 4%(w/v), and said gelatin is present in a range of from 0.1%(w/v) to 20%(w/v), preferably from 0.5%(w/v) to 20%(w/v). In one particular embodiment, the concentration of agarose in said porous polymeric matrix is 0.5% (w/v), and the concentration of gelatin is in the range of from 1% (w/v) to 2% (w/v).

In one embodiment, said label component is a mixture of at least two different dyes, preferably at least two fluorescent dyes, wherein said at least two different dyes, preferably said at least two fluorescent dyes, are present on each subset of said precursor-microparticles at a respective defined ratio and/or in defined amounts of said at least two different dyes, such that said different subsets of precursor-microparticles differ from each other in terms of the respective ratio and/or amounts of said at least two different dyes, thus allowing a distinction between said different subsets of precursor-microparticles by the respective ratios and/or amounts of said at least two different dyes attached to or contained within the respective subset of precursor-microparticles.

In one embodiment of the method of detecting and/or quantitating an analyte, said analyte of interest is a nucleic acid, said detection reaction is a nucleic acid amplification, and said detection composition is a composition for performing a nucleic acid amplification which comprises a buffer, mono-nucleoside-triphosphates, an amplification enzyme, such as a suitable nucleic acid polymerase, e.g. Taq polymerase, and a nucleic acid dye for the detection of an amplification product, such as an amplified nucleic acid, and, optionally, one or more pairs of amplification primers and, further optionally, respective molecular probes (e.g. TaqMan Probes, molecular beacons, etc.), if such primers and/or probes are not already provided as analyte-specific reagent(s) (ASR) being attached to or contained in said microparticles.

In another embodiment of the method according to the present invention, said analyte of interest is a protein or other non-nucleic acid molecule, said detection reaction is an immunochemistry detection reaction, and said detection composition is a composition for performing such immunochemistry detection reaction and is provided in said method as two separate components: wherein a first component of said detection composition comprises necessary reagents for performing an immunochemistry detection reaction, such as a buffer, and a secondary antibody or secondary antibody fragment coupled to a suitable reporter enzyme and being specific for the same analyte as a primary antibody, antibody fragment, or non-antibody protein, used as analyte-specific reagent (ASR) in said immunochemistry detection reaction; and, optionally, a primary antibody, antibody fragment, or a non-antibody protein capable of specifically binding said protein analyte or other non-nucleic acid analyte, if such a primary antibody, antibody fragment, or non-antibody protein is not already provided as analyte-specific reagent(s) (ASR) being attached to or contained in said microparticles; and wherein a second component of said detection composition comprises, as a detection reagent, a suitable substrate for said suitable reporter enzyme which substrate upon having been reacted by said reporter enzyme, becomes detectable, preferably optically detectable, more preferably detectable by fluorescence.

In such an embodiment, i.e. wherein the detection composition is provided as two separate components, namely as a first component and a second component, it is preferred that the subsets of microparticles are exposed to such second component after they have been exposed to the first component, such that the two exposures occur one after the other. The two exposures may further be separated from each other by a suitable washing step in between, e.g. a step wherein the subsets of microparticles are washed with a suitable washing buffer.

In another embodiment of the method according to the present invention, said analyte of interest is an enzyme or other clinical chemistry analyte, said detection reaction is a chemical or biochemical (e.g. enzymatic) detection reaction, and said detection composition is a composition for performing such chemical or biochemical detection reaction: wherein the sample is brought into contact with the detection reagents and both are contacted with a microparticle according to the invention, and wherein said detection composition comprises, as a detection reagent, a suitable substrate for such chemical or enzymatic creation, which substrate upon having been reacted with the respective analyte becomes detectable, preferably optically detectable, more preferably detectable by fluorescence.

In one embodiment, in said step of detecting and quantitating said analyte of interest, quantitation of said analyte is performed by a method selected from:
a) digital nucleic acid amplification, in particular digital polymerase chain reaction (PCR);
b) real-time quantitative nucleic acid amplification, in particular real-time polymerase chain reaction (PCR);
c) immunochemistry detection methods, in particular digital immunochemistry detection methods, such as digital immunoassays, e.g. digital enzyme-linked immunosorbent assays (ELISAs);
d) immunochemistry detection methods, in particular digital immunochemistry detection methods, combined with nucleic acid amplification, such as immuno-polymerase chain reaction; in particular digital immune-PCR; and
e) a combination of any of a) - d);
wherein quantitation is performed using any of methods a) or b), or a combination of a) and b), if the analyte of interest is a nucleic acid; and wherein quantitation is performed using any of methods c) or d), if the analyte is a protein, peptide or other non-nucleic acid analyte.

As already outlined above, a "biological liquid sample", as used herein, may be a liquid sample obtained from the body of an organism which may or may not have been further processed, e.g. to make analyte(s) of interest accessible or amenable to further analysis. Preferably such "biological liquid sample" is selected from blood, plasma, serum, urine, sweat, tears, sputum, lymph, semen, ascites, amniotic fluid, bile, breast milk, synovial fluid, peritoneal fluid, pericardial fluid, cerebrospinal fluid, chyle, and urine, wherein, more preferably, said biological liquid sample is plasma. Preferably such biological liquid sample is further processed, e.g. it may be lysed or digested or otherwise treated, before it is subjected to the method according to the present invention. As an example, if the biological liquid sample is plasma, such plasma may have been further lysed and/or digested to get rid of components that may otherwise interfere with the subsequent detection reaction. For example if the analyte of interest is a particular nucleic acid, the biological liquid sample, e.g. the plasma, may be first digested with a protease and other suitable enzymes to remove any unwanted proteins or peptides, as well as lipids or other unwanted components, before it is subjected to the method according to the present invention. A "biological liquid sample", as used herein, may therefore also refer to an extract obtained from any of the aforementioned bodily fluids; it may for example be a nucleic acid extract that has been obtained from any of the aforementioned bodily fluids by way of a suitable extraction, e.g. using cell disruption (if necessary), removal of lipids, proteins and unwanted nucleic acid (if necessary), and purification of nucleic acids and/or enrichment of particular nucleic acids. In some embodiments, a nucleic acid extract may have been produced using ethanol or another suitable alcohol.

Cell disruption or disintegration can be achieved by physical and/or chemical methods, whose main aim is to disrupt the cell wall and/or cellular membranes. Disruption methods are mainly based on properties of the sample and for this purpose a wide range of tools and approaches are used either alone or combined to achieve tissue/cell disruption. Lytic enzymes, chaotropic agents, and different types of detergents are the main components of chemical lysis, while mechanical method disrupts the cells by grinding, shearing, bead beating, and shocking. It should be noted that if one technique does not yield good results, another might prove successful. Osmotic shock methods have yielded, in certain cases, better results than common nucleic acid purifications protocols such as phenol-chloroform extraction and bead beating. Another approach for cell disruption is the use of different methods in combination. A good example is the case for enzymatic lysis, where many protocols use proteases to free the NA from its protective protein scaffold. Also, the inactivation of cellular nucleases that come free into solution in order to protect the new protein-free NA is crucial [13]. A combination of detergents and chaotropic salts in a single solution may be used to solubilize cell wall and or cell membrane and inactivate intracellular nucleases. Mechanical disruption, on the other hand, makes use of force to extract out constituents of the cell. A classic example of grinding is the use of mortar and pestle, which is nowadays optimized with the use of liquid nitrogen (when allowed by the sample). Cells walls can also be disrupted by the shock waves created by rapid changes in pressure elicited by sonication or cavitation. Other mechanical tools available for cell disruption are shearing, which use a tangential force to make a hole in the cell, and bead beating, which uses different glass or steel beads to rupture tough cell walls.

In a further aspect, the present invention relates to a kit for detecting and/or quantitating an analyte of interest in a plurality of biological liquid samples, in particular for performing the method according to the present invention, said kit comprising:
- a plurality of containers comprising a plurality of microparticles, each container comprising a subset of said plurality of porous microparticles, with each of said porous microparticles within each subset having a porous matrix and being configured to receive a volume of liquid in said porous matrix; each subset of microparticles being characterized by a specific label component that is attached to, contained in or otherwise associated with the respective subset, wherein, preferably, said microparticles are as defined further above; optionally, a container comprising an aqueous washing reagent for washing said microparticles;
- a container comprising a detection composition for detecting an analyte of interest; said detection composition comprising reagents for performing a chemical or biochemical detection reaction of an analyte; wherein said detection composition is either a composition for performing a nucleic acid amplification, or is a composition for performing an immunochemistry detection reaction;
- a container comprising a non-aqueous phase for transferring said different subsets of microparticles into a non-aqueous phase, once each subset has been exposed to a biological liquid sample, and for generating separate different suspensions of subsets of microparticles in a non-aqueous phase;
- a mixing container for mixing the separate different suspensions of subsets of microparticles in said non-aqueous phase together, such that all of said different suspensions of subsets of microparticles form a single suspension of different microparticles in said non-aqueous phase which is then subjected to a detection reaction;
- a container for performing a detection reaction.

In one embodiment, each of said porous microparticles has an analyte-specific reagent (ASR) attached to its porous matrix or contains an analyte-specific reagent (ASR), such analyte-specific reagent allowing an enrichment of an analyte of interest and/or allowing a specific signal or amplification reaction involving said analyte; wherein said analyte-specific reagent is capable of specifically binding to an analyte of interest.

In one embodiment, said analyte-specific reagent is selected from nucleic acids, including aptamers, Spiegelmers; antibodies or antibody fragments; non-antibody proteins capable of specifically binding an analyte or analyte complex, such as receptors, receptor fragments, and affinity proteins; wherein, preferably, said analyte-specific reagent is selected from nucleic acids, in particular nucleic acid oligomers and nucleic acid primers.

In one embodiment, said analyte of interest is a nucleic acid, said detection reaction is a nucleic acid amplification, and said detection composition is a composition for performing a nucleic acid amplification which comprises a buffer, mono-nucleoside-triphosphates, an amplification enzyme, such as a suitable nucleic acid polymerase, e.g. Taq polymerase, and a nucleic acid dye for the detection of an amplification product, such as an amplified nucleic acid, and, optionally, a pair of primers, if such pair of primers are not already provided as analyte-specific reagent(s) (ASR) being attached to or contained in said microparticles.

In one embodiment, said analyte of interest is a protein or other non-nucleic acid molecule, said detection reaction is an immunochemistry detection reaction, and said detection composition is a composition for performing such immunochemistry detection reaction and is provided in said kit in two separate compartments or containers; wherein said detection composition comprises, in a first compartment or container, necessary reagents for performing an immunochemistry detection reaction, such as a buffer, and a secondary antibody or secondary antibody fragment coupled to a suitable reporter enzyme and being specific for the same analyte as a primary antibody, antibody fragment, or non-antibody protein, used as analyte-specific reagent (ASR) in said immunochemistry reaction; and, optionally, a primary antibody, antibody fragment, or a non-antibody protein capable of specifically binding said protein analyte or other non-nucleic acid analyte, if such a primary antibody, antibody fragment, or non-antibody protein is not already provided as analyte-specific reagent(s) (ASR) being attached to or contained in said microparticles; and wherein said detection composition comprises, in a second compartment or container, as a detection reagent, a suitable substrate for said suitable reporter enzyme which substrate upon having been reacted by said reporter enzyme, becomes detectable, preferably optically detectable, more preferably detectable by fluorescence.

In one embodiment, each of said porous microparticles has the same analyte-specific reagent attached to its porous matrix or contains the same analyte-specific reagent.

In one embodiment, in said plurality of porous microparticles, there are different subsets of microparticles,
with each subset
- having its distinct label component attached to, contained in or otherwise associated with said microparticles of said subset; and
   all of said different subsets having
- the same analyte-specific reagent attached to or contained in said microparticles of said subsets, said analyte-specific reagent being specific for one analyte of interest;
   such that said different subsets of microparticles are identical in terms of the analyte-specific reagent attached or contained, but differ by
- the respective label component attached to, contained in or otherwise associated with said microparticles of each subset;
   with each subset being unambiguously defined and identifiable by said respective label component and being provided in a separate container.

In one embodiment, said kit is a kit for detecting and/or quantitating one (i.e. not more than one) analyte of interest in a plurality of biological liquid samples, wherein the number of different subsets of microparticles provided in said kit equals (preferably at least equals) the number of separate biological liquid samples provided (or in some embodiments the number of different subsets of microparticles provided in said kit is greater than, and in any case is not smaller than, the number of separate biological liquid samples provided).

In another embodiment, in said plurality of porous microparticles, there are several different analyte-specific reagents attached to or contained in said microparticles.

In such an embodiment, there are different subsets of microparticles,
with each subset
- having its distinct label component attached to, contained in or otherwise associated with said microparticles of said subset; and
   wherein furthermore, in said plurality of porous microparticles, there are different classes of subsets of microparticles with each class of subsets
- having a different analyte-specific reagent attached to the porous matrix of said microparticles or contained in said microparticles; wherein, preferably, there are at least two different classes of subsets of microparticles, more preferably at least three or more different classes of subsets of microparticles;
such that said different subsets of microparticles differ by the respective label component attached to, contained in or otherwise associated with said microparticles of each subset; and each subset of microparticles forms part of one class of subsets of microparticles; with each subset being unambiguously defined and identifiable by the respective label component and the respective analyte-specific reagent and being provided in a separate container; and
such that said different classes of subsets of microparticles differ by the respective analyte-specific reagent attached to the porous matrix of said microparticles or contained in said microparticles; and each of said different classes comprises several subsets of microparticles, all of which subsets have the same analyte-specific reagent attached or contained.

In such an embodiment, said kit is a kit for detecting more than one analyte of interest in a plurality of biological liquid samples, wherein, the number of different subsets of microparticles provided in said kit equals (preferably at least equals) the number of separate biological liquid samples provided, multiplied by the number of analytes of interest to be detected, and wherein, in said kit, there are as many (preferably at least as many) classes of subsets of microparticles provided as the number of analytes of interest to be detected; (or in some embodiments the number of different subsets of microparticles provided in said kit may, in fact, even be greater than, and in any case is not smaller than, the number of separate biological liquid samples provided, multiplied by the number of analytes of interest to be detected; and wherein, in such an embodiment of kit, there are at least as many classes of subsets of microparticles provided as the number of analytes of interest to be detected, and in any case the number of classes of subsets of microparticles provided in such embodiment is not smaller than the number of analytes of interest to be detected).

In a further aspect, the present invention also relates to a cartridge for performing a method of detecting and/or quantitating an analyte of interest in a plurality of biological liquid samples, said method being as defined further above.

In one embodiment, the cartridge comprises a plurality of sample-specific modules, a plurality of storage chambers, at least one non-aqueous phase chamber for storing a non-aqueous phase, and either a single combined mixing and detection chamber, or a combination of a separate mixing chamber and a separate detection chamber;
wherein each sample-specific module comprises a sample compartment having its own separate sample inlet, each sample-specific module being configured to separately receive exactly one biological sample only, in the respective sample compartment; each sample-specific module being furthermore configured to receive microparticles in said sample compartment, said microparticles being as defined further above; each sample-specific module being further configured to facilitate a phase-transfer of said microparticles from an aqueous environment to a non-aqueous environment.

The term "sample-specific module" is meant to refer to a section within the cartridge that is specifically designated to be used in conjunction with a particular (specific) sample. As such it typically comprises a sample compartment and is configured to separately receive exactly one biological sample only, in the respective sample compartment. A "sample-specific module" has its own specific sample inlet, allowing the addition of the respective sample to the sample-specific module. may additionally comprise further compartments and/or connecting channels. In one embodiment, said plurality of sample-specific modules comprise a plurality of porous microparticles in the respective sample compartments; each of said porous microparticles having a porous matrix and being configured to receive a volume of liquid in said porous matrix; wherein each sample-specific module in its sample compartment comprises a different subset of said plurality of microparticles, each subset of microparticles being characterized by a specific label component that is attached to, contained in or otherwise associated with the respective subset; and/or wherein said at least one non-aqueous phase chamber comprises a non-aqueous phase, such as an oil..

In one embodiment, each of said sample-specific modules further comprises a means for mechanical separation of microparticles from a liquid phase, preferably a liquid aqueous phase, such as a filter; said means being configured to facilitate a phase-transfer of said microparticles from an aqueous environment to a non-aqueous environment.

In one embodiment, in said plurality of storage chambers there are different groups of storage chambers, with a first group of storage chambers containing reagents for performing a chemical or biochemical detection reaction of an analyte and, separately, with one or several further groups of storage chambers separately comprising one or several of: a lysis buffer, a buffer for facilitating the binding of an analyte to an analyte-specific reagent, and a washing buffer for washing microparticles.

In one embodiment, the cartridge comprises a valve mechanism, said valve mechanism being configured to be attached to a pump, said valve mechanism allowing to bring serially or concomitantly into fluid connection any of a) to c), with d); wherein a) is one or several of the storage chambers; b) is either the single combined mixing and detection chamber, or the combination of a separate mixing chamber and a separate detection chamber; c) is said at least one non-aqueous phase chamber; and d) is any of the sample-specific modules..

The present inventors have provided for a method of detecting and/or quantitating an analyte of interest in a plurality of biological liquid samples. Without wishing to be bound by any theory, the present inventors believe that the new method according to the present invention is based on a new assay in principle that comprises two fundamentally different parts, namely a sample-specific part and a generic part (herein also sometimes referred to as "specific process part" or "specific part", and "generic process part", respectively).

In the sample-specific part of the process, individual samples are encoded and the analyte, if present in individual samples, is enriched, whilst at the same time undesired materials are removed from the sample. Thereafter, the thus encoded sample that has been made identifiable by its individual code/label, is insulated as a kind of aqueous droplet defined by the microparticle in a non-aqueous environment. Such droplet acts as the reaction space in which the detection of the analyte, if present, is performed.

Generally speaking, the aforementioned aim is achieved by interrogating (and effectively "labelling" or "encoding") each sample individually with a specific and individually labeled type or subset of microparticle, respectively, a "microparticle" herein also sometimes being referred to as "bead", which specific and individually labeled type or subset of microparticle carries a defined specific label that is attached to, contained in or otherwise associated with the respective microparticle. In accordance with embodiments of the present invention, there may be a plurality of different types of microparticles (or "beads") (such "types of microparticles" herein also sometimes referred to as "subsets of microparticles"), which are characterized by the specific label attached thereto, contained therein or otherwise associated therewith. In accordance with embodiments of the present invention, the microparticles are able to receive a volume of liquid sample and thereby take up the analyte, if present in the sample, and optionally also bind and concentrate it. Binding of an analyte to microparticles may be facilitated by exposure of microparticles to a sample suspected of containing an analyte of interest in the presence of a binding buffer. Such binding buffer serves the purpose of establishing conditions that promote the binding of the analyte to the matrix of the respective microparticles and/or any analyte-specific reagent (ASR) attached to or contained in the microparticles.

Furthermore, in accordance with embodiments of the present invention, the individual microparticles have an internal volume allowing them to take up the sample and necessary reagents for detecting an analyte of interest, if present. Once the analyte in sample has been taken up (or "absorbed") and, possibly, bound or enriched (or "adsorbed") by the microparticle, optionally in the presence of s suitable binding buffer, and once any respective washing steps, if necessary, have been performed, the microparticle is subsequently exposed to an appropriate detection composition that will also be taken up by the microparticle. The term "detection composition", as used herein refers to a composition comprising reagents for performing a chemical or biochemical detection reaction of an analyte. In preferred embodiments, the "chemical or biochemical detection reaction" is a nucleic acid amplification or an immunochemistry detection reaction. Suitable "detection compositions" for performing such reactions are outlined further above. Once such detection composition has been taken up by the microparticle, there is thus a microparticle that contains a biological liquid sample (or a portion of such sample) and a suitable detection composition. Subsequently, any remaining liquid not contained within the microparticle, but situated outside of the microparticle, will be removed, for example by filtration, centrifugation, shaking or other mechanical agitation, and combinations thereof, and the respective microparticles are then insulated by placing them into a non-aqueous liquid which insulates the respective microparticles and prevents them from interacting with each other. Such a process is repeated on multiple samples, with each sample being interrogated by a different specific subset of microparticle, specifically labeled by a specific label component. The result of this process are separate subset microparticle preparations, i. e. separate subset preparations with individual microparticles within each subset being separated from each other, and with individual subset microparticles' preparations having their respective individual label component assigned thereto, thus allowing such subset microparticle preparation to be unambiguously assigned to a specific sample.

At this stage, i. e. after the microparticles have been insulated, different types of microparticles' preparations, i.e. different subsets, corresponding to different samples, may be mixed and pooled together. Also at this stage, because the individual microparticles have been insulated and can no longer interact with each other, because they are individual aqueous droplets encompassed by non-aqueous phase, different types of microparticles' preparations corresponding to different samples to which they have been exposed, may be combined into a pool, and such pool may subsequently then be subjected to conditions that are necessary to perform a detection reaction within the microparticles. Such a detection reaction may, for example, be a nucleic acid amplification, or it may be an immunochemistry reaction. Such detection reaction represents the aforementioned generic part of the method. It is "generic", because it is performed on any of the microparticles, i. e. any type of microparticles, as long as such microparticle has been interrogated by a sample suspected of an analyte. In other words, such generic part is performed on all microparticles, preferably simultaneously as a single process, irrespective of the type of sample to which such microparticles have been exposed.

The signal generated within each microparticle, as a result of the detection reaction, is detected and assigned to the respective specific label component that is attached to, contained in or otherwise associated with the respective microparticle. Because it is also known which label component corresponds to which sample, the respective signal that has been generated can, effectively, be correlated with the respective sample, as a result of which it can be determined whether or not the analyte of interest is or was present in the sample to be tested.

Effectively, the microparticles represent individual reaction spaces or "reactors" in which minute quantities of a respective sample have been confined and may be individually analyzed. Therefore, it is effectively possible to perform embodiments of the method in accordance with the present invention in such a manner that single molecules of analyte can be distributed in single microparticles, thus providing for a limited dilution and thus allowing the method to be performed in a digital format; see also Sykes et al., 1992, Biotechniques 13(3): pp. 444-449. Such a format allows for an exquisite sensitivity and a good quantification of analyte in the respective sample.

Typical reagents that are used in embodiments of the present invention are as follows:
First of all, there are the microparticles themselves, herein also sometimes referred to as "beads". These are highly porous and have a porous matrix and are configured to receive a volume of liquid in the porous matrix. Typically, they are composed of a polymer or a polymer mixture providing for mechanical stability and chemical integrity of the microparticle when the liquid sample is taken up and is accommodated in the porous matrix. Preferably the matrix polymer(s) may exhibit gel-sol characteristics providing for stable particles at room temperature and for droplets at elevated temperatures. As an example, if the microparticles are in their gel-state, i.e. a gel or quasi-solid phase, they will form a suspension in a liquid phase. However, if subsequently, they are converted into a sol state, i.e. a soluble or quasi-liquid state, they will form an emulsion in a liquid phase, and the suspension will transform into an emulsion.

The term "microparticle", as used herein, is meant to refer to a particle the average dimensions of which are in the micrometer range. In one embodiment, the microparticles in accordance with the present invention have an average size or average dimension or average diameter of approximately 1 µm - 200 µm, preferably 5 µm - 150 µm, more preferably 10 µm - 100 µm. In one embodiment, the microparticles in accordance with the present invention are spherical or oval or ellipsoidal, preferably spherical, and the above-mentioned dimensions refer to the average diameter of such spherical, oval or ellipsoidal microparticle. In one embodiment, the microparticles have the shape of a (spherical) droplet. In another embodiment, a microparticle in accordance with the present invention is a spherical body or a quasi-spherical body, i. e. having the shape of a sphere (or nearly approaching it), such sphere having an average diameter of the aforementioned dimensions. Typically, microparticles in accordance with the present invention are porous and have a porous polymeric matrix having a void volume for receiving an aqueous sample and for providing a reaction space for the specific detection of an analyte.

In preferred embodiments, the microparticles according to the present invention are not only capable of taking up a liquid sample and accommodating the same, together with any analyte that is present in such sample, within the porous matrix, but actually comprise a binding member (e.g. a analyte specific binding molecule attached to said porous polymer matrix, e.g. an analyte-specific reagent (ASR); ionizable groups, or a plurality of ionizable groups, immobilized on said porous polymer matrix, said ionizable group(s) being capable of changing its(their) charge(s) according to ambient conditions surrounding said precursor-microparticle; a charged group, or a plurality of charged groups immobilized on said porous polymer matrix; a combination of any of those) that facilitates or provides the required functionality to bind or enrich an analyte or analyte class (e.g. nucleic acids) of interest, if present in a sample, and thereby increase the local concentration of such analyte within the microparticle. Furthermore, in preferred embodiments of the present invention, a microparticle is further characterized by a specific label that is attached to, contained in or otherwise associated with the respective (type of) microparticle.
It has turned out that microparticles, such as are disclosed in the parallel EP application entitled "A library of prefabricated microparticles and precursors thereof'
(attorney docket B33016EP) by the present applicant Blink AG, are particularly suitable also in embodiments of the present invention, in particular in such embodiments where more than one analyte is to be detected in each sample.
In some embodiments, a lysis buffer may be required to release the analyte of interest from the sample or make such analyte of interest available within the sample. Such a lysis buffer may contain an agent that may cause a lysis of cells, in particular cell membranes, or a lysis of cellular organelles, as a result of which lysis, an analyte of interest may only become accessible. In some embodiments, a lysis buffer alone or in combination with a further buffer may facilitate the enrichment or binding of an analyte of interest in a microparticle. Such a further buffer that facilitates the enrichment or binding of an analyte of interest in a microparticle is herein also sometimes referred to as a "binding buffer" If such "binding buffer" is used to adapt the analyte's or other component's concentrations, such "binding buffer" is herein also sometimes referred to as "dilution buffer".

The term "analyte of interest" or "analyte", as used herein, is also herein sometimes referred to as "target", in particular if nucleic acids are being analyzed.

In some embodiments, also a wash buffer may be required to remove any undesired component from the microparticle(s) which would otherwise inhibit a subsequent detection reaction or which would otherwise prevent such subsequent detection reaction to function properly. If, for example, the detection reaction is an immunochemistry reaction, in some embodiments, the microparticle(s) may comprise an analyte-specific reagent which is a primary antibody that is specific for the analyte of interest. In such an embodiment, the microparticle(s) including the primary antibody as analyte-specific reagent, is exposed to a sample that is suspected of containing an analyte of interest, and such microparticle is subsequently exposed to a first detection composition comprising a secondary antibody that is specific for the analyte of interest bound to the primary antibody and that is, itself, coupled to a suitable reporter, e. g. an enzyme. Once the microparticle has been exposed to the sample and to the first detection composition, it may be necessary to perform a washing step in order to remove any unbound secondary antibody that might otherwise interfere with the subsequent detection reaction. Hence, embodiments of the present invention envisage the use of a washing step involving a wash buffer to remove any undesired component from the microparticles that may subsequently inhibit or prevent a proper reaction detection reaction to take place.

In a further step in one embodiment, the microparticle(s) is (are) exposed to a second detection composition which comprises, as a detection reagent a suitable substrate for the reporter enzyme that is attached to the secondary antibody. This substrate, upon having been reacted by said reporter enzyme, becomes detectable, preferably optically detectable, in particularly detectable by fluorescence.

In other embodiments of the invention, the detection reaction may be nucleic acid amplification reaction. In such embodiments, the microparticle(s) may comprise oligonucleotide primers and probes for target specific amplification and detection. Such analyte-specific reagent(s) may be already provided reversibly bound to the microparticle(s) or may be added thereto together with other components of a generic detection composition. Typically, such generic detection composition comprises reagents necessary for performing an amplification reaction of the nucleic acid analyte of interest and may, or may not, also contain primers and, optionally, suitable detection probes, depending on whether these primers and/or probes are already part of the microparticle(s), or not. If such analyte-specific reagents are already part of the microparticle(s), they will not be comprised in the generic detection composition. However, such generic detection composition otherwise comprises reagents necessary for performing amplification reaction of the nucleic analyte of interest, except for primers, wherein, in particular, the generic detection composition comprises a suitable buffer, mono-nucleotides, an amplification enzyme, such as a suitable nucleic acid polymerase, e. g. Taq polymerase, and (optionally) a nucleic acid dye for the detection of an amplification product, such as an amplified nucleic acid.

According to the present invention, the method also requires a transfer of the microparticle(s) to a non-aqueous phase which is used to insulate the individual microparticles, thereby effectively sealing and confining their contents to the volume of the microparticle(s). Such a non-aqueous phase typically is a lipophilic compound, e. g. a lipophilic oil, such as a fluorocarbon oil which, preferably, contains one or several suitable emulsifiers. Such a non-aqueous phase is required when the microparticle(s) are transferred into it, and such non-aqueous phase helps to displace any aqueous liquid that is not taken up by the microparticle(s) and to isolate such microparticle(s) from each other such that no liquid exchange is possible anymore between different microparticles. As a consequence, once a stable microparticle suspension is formed any sample carry-over from one microparticle to another, which may lead to a mixing of two different samples, is thus excluded.

In addition, embodiments of the method according to the present invention enable an elegant approach for analyte quantification by bridging quantitative digital and quantitative real time analysis approaches. While this statement is true for any kind of signal or target amplification assay being employed on the microparticles, PCR amplification may serve as an example to illustrate the approach to quantitation of targets with our method. Quantitation of target molecules is preferably conducted by digital PCR (dPCR) because this method allows more sensitive and precise quantitation than real-time quantitative PCR (qPCR). A disadvantage of digital PCR is the inherent limitation of the measuring range that depends on the number of microparticles specific for one sample/analyte. To overcome this limitation, the invention supplements the analysis of digital PCR with real-time quantitative PCR for target concentrations above the digital PCR measuring range. The implementation of this approach requires acquisition of fluorescence images at the end of PCR and during all cycles of the real-time PCR. All acquired images are analysed by an image analysis algorithm in order to quantify fluorescence level of each microparticle in the reactor chamber. A segmentation algorithm separates bright disk-shaped microparticle objects from dark background. Based on this segmentation information, finally circles are fitted to microparticle object contours allowing estimation of mean microparticle fluorescence and microparticle volume. The analysis of real-time PCR images includes tracking of microparticle positions in consecutive images to allow monitoring fluorescence during the course of the reaction in each respective microparticle.
The selection of the applied quantitation approach is based on the number/proportion of microparticles remaining negative after amplification reaction. This information is taken from digital PCR data. If a predefined lower limit for number/proportion of negative microparticles is exceeded, the end point Poisson analysis can be applied. Otherwise real-time analysis is conducted using real-time quantitative PCR data acquired during all cycles. A reasonable lower limit for the proportion of negative microparticles allowing still robust quantification with Poisson is 0.5%. Corresponding mean number of targets per microparticle is 5.3. To consider possible artefacts on fluorescence images an additional requirement can be a lower limit for the total number of negative microparticles of e.g. 50.
The end point Poisson analysis is performed by determining the proportion of negative microparticles and applying a Poisson correction to account for the fact that positive microparticles can contain more than one target molecule. The threshold, distinguishing positive and negative microparticles, is directly estimated from fluorescence signal intensities of microparticles known to be negative. Possible microparticle volume variations are factored into the quantitation by performing microparticle volume specific Poisson corrections. Possible variations of the total microparticle volume between measurements are also corrected by the algorithm.
If target concentration exceeds the measuring range of digital PCR, real-time analysis fits a nonlinear function to the fluorescence signal course of each single microparticle. The fitted nonlinear model combines a sigmoid and a linear function where the sigmoid component reveals amplification kinetics and the linear component represents baseline of the signal. The cycle threshold value (Ct) is calculated from the intersection of tangent in definition value of sigmoid function with maximum second derivation and baseline. Respective target numbers per microparticle are calculated using a calibration data set. The offset term of the calibration curve can be a function of the microparticle volume to account for the effect of microparticle volume variations.
A simple rule based on the number of microparticles remaining negative (i.e. "dark") after a signal or target amplification reaction has been performed in the reaction spaces created by the microparticles, can be applied to establish which approach is best suited to achieve quantitative results over a broad measurement range. The rule can be formulated as such:
a. if, for a given detection/quantification measurement using a library according to the present invention, more than 0.5% of microparticles specific for a given analyte remain negative (i.e. the probability of a microparticle ("bead") remaining dark ("PdarkBead" or "Pnegative") is >0.5%), Poisson Analysis can be applied to determine the concentration of target analytes in the sample.
b. if, for a given detection/quantification measurement using a library according to the present invention, less than 5% of microparticles specific for a given analyte remain negative (i.e. the probability of a microparticle ("bead") remaining dark ("PdarkBead" or "Pnegative") is <5%), real time analysis algorithms can be applied to determine the concentration of target analytes in the sample
c. if, for a given detection/quantification measurement using a library according to the present invention, the "negative" microparticles occur within a range of 0.5%< Pnegative <5% both approaches can be applied interchangeably.

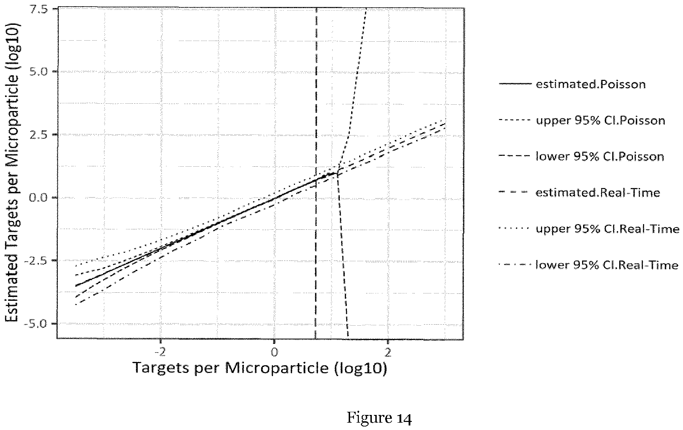

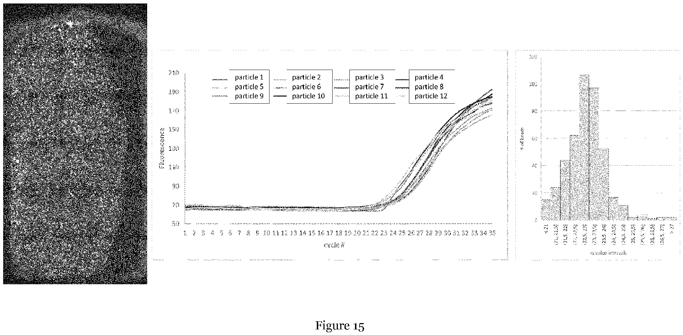

This principle of quantification is also illustrated in Figures 14 and 15 as well as in Example 2.

Furthermore, reference is made to the figures which are given to illustrate, not to limit the present invention. More specifically,

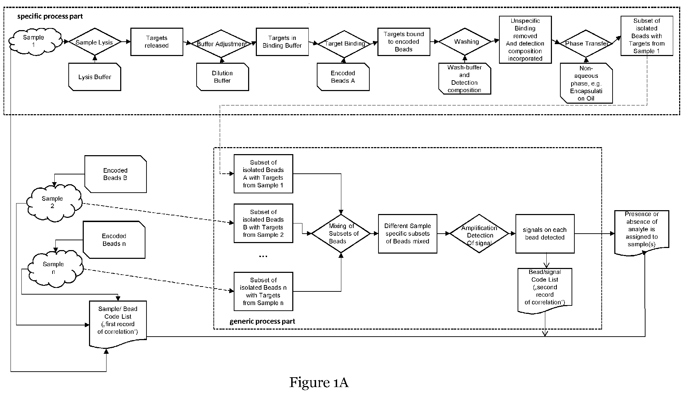

**Figure 1A** shows an embodiment of a basic scheme of an assay/method in accordance with embodiments of the present invention showing that the workflow of such method comprises a sample-specific part (referred to in the figure as "specific process part") that is preferably performed for as many times as there are different samples to be tested, and a generic process part which is common to all microparticles, irrespective of which sample(s) they have been exposed to. The upper box depicts an embodiment of a basic process and possible components of a sample-specific part of the process. A sample is lysed using a lysis buffer, as a result of which the analyte(s) of interest, herein also sometimes referred to as "target(s)", is (are) released. Optionally, a suitable dilution buffer (herein also sometimes referred to as "binding buffer") may be used to adjust concentrations of certain reagents in the lysis buffer to an acceptable range, whereupon the sample including the suspected analyte of interest, is subjected to conditions in which the sample including the analyte(s) of interest, may be absorbed or adsorbed by microparticles that carry a certain label that is now assigned to the respective sample, such labelled microparticles herein also sometimes referred to as "encoded microparticles" or "encoded beads". Such "encoded microparticles" that have been exposed to one sample, are herein also sometimes referred to as a "subset of microparticles". Each such "subset" is characterized by a specific label component that is attached to, contained in or otherwise associated with the respective subset. In some embodiments, the respective analyte may also be bound to the microparticle(s) by affinity binding to the matrix forming the particle or to an analyte-specific reagent (ASR) that have been attached to or are contained in the particles for this purpose. Once the analyte(s) of interest has been incorporated/accommodated within the microparticle(s), the respective subset of microparticles is exposed to a suitable detection composition the nature and content of which depends on the detection reaction that is to be performed with the analyte of interest (which detection reaction, in turn, depends on the type of analyte to be detected). Such detection reaction comprises reagents necessary for performing a detection reaction of the analyte of interest. Preferred examples of such detection composition are further defined above. Subsequently, once the respective detection composition has been taken up in the microparticles, there may follow a step of removing any aqueous phase that is surrounding the respective microparticle(s). Such step may involve filtration, centrifugation, shaking or other mechanical agitation, and combinations thereof, that is performed with the respective microparticles. Thereafter, preferably after removal of any aqueous phase surrounding the respective microparticle(s), the respective microparticle(s) are transferred into a non-aqueous phase, such as an oil, optionally including an emulsifier. In preferred embodiments, the emulsifier helps in isolating the individual microparticle. As a result of such transfer into a non-aqueous phase, there will be a suspension of isolated microparticles with, possibly, analyte(s) of interest from this particular sample. The same process is repeated with a different type ("subset") of microparticles, encoded differently, i. e. having a different label component attached to, contained in or otherwise associated therewith, with such differently encoded microparticles being exposed to a different sample. Once this process has been performed for as many different samples as necessary or desired, a first record of correlation may be generated indicating which separate subset of microparticles has been exposed to which sample. Such first record of correlation is herein also sometimes referred to as "sample/bead code list". Once the process of exposure of different subsets of microparticles to different samples has been performed for as many different samples as desired or necessary, the respective isolated different subsets of microparticles may be mixed and subjected in the generic part of the process to a detection reaction which may be a nucleic acid amplification, or the performance of a immunochemistry reaction, or any other bio-chemical reaction suitable for detection an analyte of interest. Depending on the presence or absence of analyte in the respective sample (and subset of microparticles), there may be a signal generated in such detection reaction for a particular subset of microparticles. A list of for which beads a signal is detected ("Beads/signal code list" or "second record of correlation") may thus be generated indicating in which subset of microparticles a signal has been generated in the detection reaction. Subsequently, the respective signal(s) generated in such detection reaction may subsequently be decoded (e.g. by aligning or comparing the second record of correlation (or the "Beads/signal code list") with the first record of correlation (or the "sample/bead code list"), resulting in the finding that the analyte(s) of interest has been present (or absent) in one or several of the samples that had originally been used to fill/load the respective microparticle(s). Preferably, the determination of which sample contains the analyte of interest, i.e. the actual assignment of detected signal to is performed by linking the first record of correlation (i.e. the list indicating which subset has been exposed to which sample) with the second record of correlation (i.e. the list indicating in which subset a signal has been generated).
   It should be noted that a major advantage of the method according to the present invention is the following: If samples were to be tested individually, a detection reaction would have to be performed individually and separately for each sample. The present invention allows for one part of the method (the "generic part") which is identical and common to all the detection reactions that would otherwise be performed individually and separately for each sample, to be performed once for all samples being analyzed.

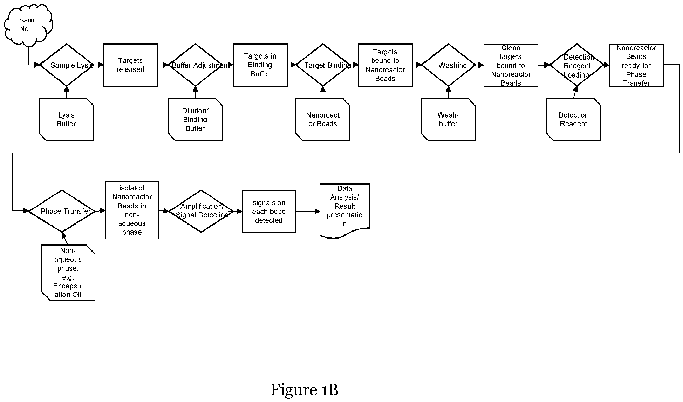

**Figure 1B** shows an embodiment of a basic scheme of an assay/method in accordance with embodiments of the present invention showing that the workflow of such method can be also applied favorably for processing a single sample. The employed beads provide for a lean process that utilizes the porous material for target enrichment, clean-up and for performing a signal generation reaction such as PCR in individual nanoreactors, that provide for exquisite quantitation of target present in the sample across a broad measurement range.

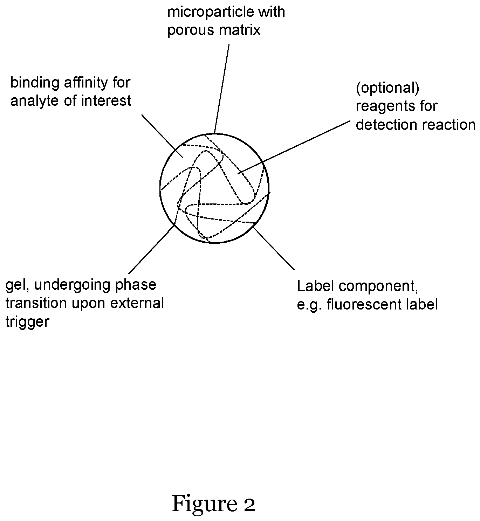

**Figure 2** shows an embodiment of an exemplary microparticle in accordance with the present invention which has a porous matrix and is made up of a polymer which, in this particular case, is also capable of undergoing a phase transition upon an external trigger. For example, in a preferred embodiment, the microparticle having a porous matrix is composed of a porous polymeric matrix which polymer making up such porous polymeric matrix is capable of under undergoing a sol-gel transition, for example upon raising the temperature. Once the respective microparticles have been insulated in a non-aqueous phase, the temperature may be raised up to a level, where the microparticle undergoes such phase transition, resulting effectively in the formation of an aqueous droplet in which a detection reaction may take place. In accordance with this embodiment shown in figure 2, the microparticle(s) also has an increased binding affinity for a particular analyte of interest. This may be a feature of the polymer matrix itself or, for example, be achieved by an analyte-specific binding molecule (binder) reagent attached to the matrix. Such binder may, in some instances, for example, be an analyte-specific reagent (ASR), as defined further above. Analyte-specific binders may be selected from nucleic acids, including nucleic acid pairs, in particular primer pairs; aptamers, Spiegelmers; antibodies or antibody fragments; non-antibody proteins capable of specifically binding an analyte or analyte complex, such as receptors, receptor fragments, and affinity proteins. In a preferred embodiment, the analyte-specific reagent is selected from nucleic acid, in particular nucleic acid oligomers and nucleic acid primers. Furthermore, the microparticle(s) has (have) a label component attached to or contained within or otherwise associates therewith. Moreover such a microparticle may (optionally) comprise reversibly attached reagents for a signal generating detection reaction to be performed within the space provided the microparticle. It should be noted that, in accordance with embodiments of the present invention, a pair of primers that is suitable for amplifying a particular nucleic acid sequence (which is the "analyte" or "target"), may herein qualify and be regarded as a single analyte-specific reagent (ASR) despite the fact that such pair of primers comprises two different primers. They are, however, specific for a single nucleic acid sequence in that they flank the same region of such nucleic acid sequence, and thus are specific for the same nucleic acid analyte
   In preferred embodiments, the label component is a fluorescent dye, more preferably a mixture of two different fluorescent dyes.
   The microparticle(s) in accordance with embodiments of the present invention possess a capacity to accommodate, preferably bind or enrich, the analyte of interest in their porous matrix, and can be identified and distinguished against other microparticles by means of the respective label component that is attached to, contained in or otherwise associated with the respective microparticle(s). Because the microparticle(s) in accordance with the present invention is (are) of a porous nature, they provide for an accessible inner volume that is sufficient to take up (or "absorb") sample, including any analyte of interest, if present, or to bind (to "adsorb") the analyte to the matrix of the microparticle by e.g. an analyte specific binding molecule attached to said porous polymer matrix; ionizable groups, or a plurality of ionizable groups, immobilized on said porous polymer matrix, said ionizable group(s) being capable of changing its(their) charge(s) according to ambient conditions surrounding said precursor-microparticle; a charged group, or a plurality of charged groups immobilized on said porous polymer matrix; or a combination of any of those.
   Preferably, such microparticles are furthermore capable of undergoing a phase transition upon the application of an external trigger, such that they may, for example upon raising the temperature above a certain threshold, transform from a gel state at room temperature into a soluble state at elevated temperature. When the particles at the same time are in a liquid, their suspension at room temperature will, thus, transform into an emulsion at elevated temperatures.
   Preferred polymers that function in this manner are agarose polymers, alone or in conjunction with gelatin.

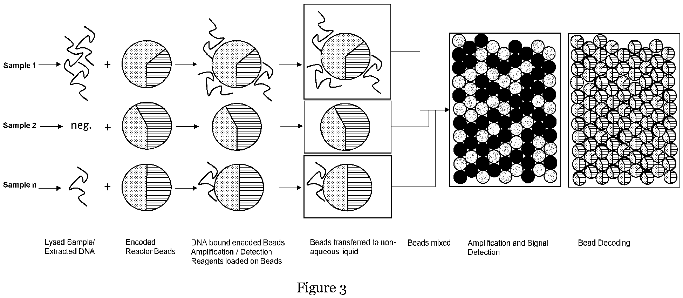

**Figure 3** shows the basic functionality of the microparticles ("Reactor beads") in accordance with embodiments of the present invention. The figure shows three different types of microparticles, each being distinguished from each other by virtue of the respective label component associated with such respective type. In the present representation of figure 3, the different label components are symbolically represented by differently hatched areas within the circular representations of the microparticles/beads; such differently hatched areas may stand for any suitable label components as long as they allow to distinguish the different microparticles. E.g. they may represent different concentrations of a single dye, different ratios of two different dyes, different detectable physical labels, etc. In a first part of the process which is the "sample-specific part" of the process, the three different types of microparticles are separately exposed to three different samples 1 - 3, such that, effectively, each sample is encoded by the label component of the respective type ("subset") of microparticle which is exposed to such sample. In a preferred embodiment, such correlation between label component and respective sample associated therewith, is achieved by generating a list of label components (of corresponding subsets of microparticles) with corresponding associated samples, herein also sometimes referred to as "a first record of correlation". Such a list may herein also sometimes be referred to as a "sample/microparticle code list" or "sample/bead code list", indicating which subset of microparticles has been exposed to which sample. In this embodiment, following a series of process steps, the microparticles are loaded with detection reagents and are collected in a non-aqueous environment that provides for an insulation of the microparticles and prevents any cross talk between the microparticles. In a second part of the process, which is the "generic part" of the process, the different suspensions of microparticles that encode and correspond to different samples in a non-aqueous phase are mixed and are subjected simultaneously ("in one go", i.e. no longer separately from each other) to conditions that are necessary to perform the required detection reaction, for example a nucleic acid amplification, such as PCR involving a temperature cycling. Because such "generic part" allows the processing of a large number of samples, this facilitates the performance of a single detection process with samples of entire patient cohorts of a large size, e.g. in a clinical trial. The signal(s) produced in such detection reaction is(are) recorded, and the respective label components of the (subsets of) microparticles are determined/read. A second record of correlation is generated indicating in which subset of microparticles a signal has been generated. Such second record of correlation is herein also sometimes referred to as "Bead/signal code list"). The resultant detection signal/microparticle combinations in the "bead/signal code list" (i.e. the second record of correlation) are compared with the "sample/microparticle code list" ("i.e. the first record of correlation), as a result of which it can be determined which sample (of the plurality of tested samples) did or did not contain the respective analyte of interest.
   Microparticles which are particularly suitable for embodiments, in accordance with the present invention are the microparticles as contained in a library of prefabricated microparticles for performing a specific detection of an analyte of interest in a sample, as described in co-pending European patent application entitled a library of prefabricated microparticles (Attorney docket B33016EP), filed concurrently herewith.

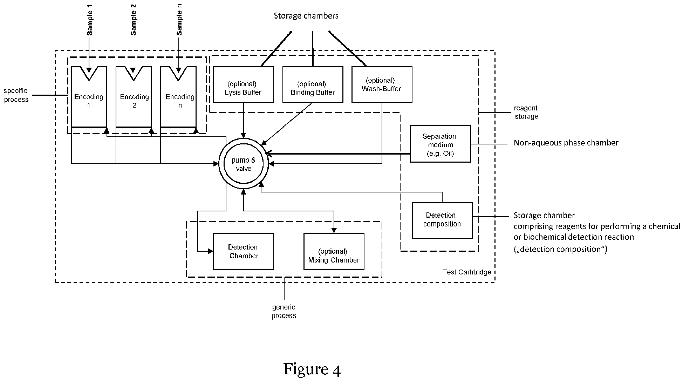

**Figure 4** shows a schematic diagram of an exemplary layout of a novel fluidic cartridge that may be suitable for performing embodiments of the method according to the present invention. Unique features of the cartridge are separate multiple inlets for the individual samples to be analyzed. Each inlet is separately connected with its own corresponding sample chamber or sample-specific module, and there are a number of different sample chambers or sample-specific modules provided in the cartridge each of which is intended and configured to be used for a separate sample. The terms "sample chamber", "sample chamber module" and "sample-specific module" are used interchangeably herein and refer to a chamber that is specifically intended and configured to receive a specific sample. The actual space within the respective sample-specific module that is configured to contain the sample (and to contain the specific subset of microparticles for this particular sample) is herein also sometimes referred to as "sample compartment". These specific chamber modules are provided to ensure a safe encoding process, i. e. a process in which each sample becomes encoded/labelled by exposing a specific separate type ("subset") of microparticles to such sample, thus resulting in each of said different subsets of microparticles being "loaded" with a different sample, respectively. The different sample-specific chamber modules prevent any cross contamination between the different modules and thus different samples. The respective separate inlets are shown in the figure by triangular indentations in the sample chambers (or sample-specific modules) which are represented by rectangular boxes standing on their smaller side(s).
   The exemplary cartridge layout in this figure comprises an exemplary valve and pump mechanism. Three separate groups of chambers (or modules) are provided on the cartridge layout:
   1. modules facilitating the sample specific process and that among other parts contain the sample encoding microparticles
   2. chambers for storing regents required to perform the specific (if not provided within the sample specific modules) and the generic processes; also shown is a specifically designated storage chamber for containing a non-aqueous phase ("non-aqueous phase chamber")
   3. chambers or modules facilitating the generic process (e.g. a mixing (optional) and a detection (compulsory) chamber, or a combined mixing and detection chamber, i.e. a chamber wherein both functions are combined (not shown))
   All the reagents are delivered to the respective modules in a unidirectional fashion before the encoding process has been completed, and before the microparticles are insulated, i. e. secured in the non-aqueous phase. Eventually, a suspension of microparticles is collected from each sample module, and the different suspensions are then mixed together to form a single suspension. Such mixed microparticles are then transferred to a detection chamber and are subjected to the necessary conditions that are required for performing a detection reaction. A cartridge that may be adapted and, upon modification, be used in accordance with embodiments of the present invention is a cartridge as described in European patent application EP No. 19 187 064.1, filed on July 18, 2019, which needs to be adapted however, insofar as it requires modification in that the cartridge according to the present invention has a plurality of sample chambers, and each of such sample chambers has its own sample inlet. This allows for the separate manipulation of a plurality of samples each of which becomes separately labelled in accordance with the present invention.

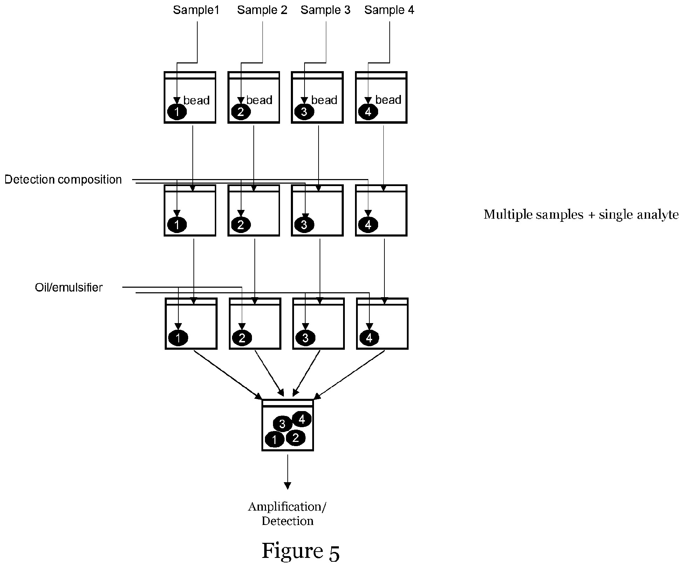

**Figure 5** shows an embodiment of a method according to the present invention. More specifically, different subsets of microparticles (referred to in the figure as "bead 1", "bead 2", "bead 3" and "bead 4", respectively) have a different label component attached, but either contain no analyte-specific reagent at all, or they comprise, exactly, one type of analyte-specific reagent that is specific for one specific analyte of interest. The different subsets of microparticles are identifiable by their different label components and are provided separately, e.g in different containers, such as different sample chambers or sample-specific modules within a cartridge, as described further above, and each of them is exposed separately to a different sample. Effectively, there will be used as many subsets of microparticles as there are samples to be tested. Thereafter, the necessary (generic) detection reagent/reagent composition is added to each subset (with such detection composition optionally additionally also including an analyte-specific reagent, as defined further above, if such analyte-specific reagent had not been contained within the microparticles from the start). Then, the respective subsets of microparticles are, still separately, transferred into a non-aqueous phase (which may be an oil, optionally containing emulsifier), thereby becoming insulated from each other, as a result of which there will be no "cross talk", i. e. mixing of samples between the spaces provided by the respective microparticles, anymore, and thereafter a (generic) detection reaction is performed ("one-pot detection reaction"). The results of such (generic) detection reaction allows for a conclusion to be drawn in respect of which sample the one analyte of interest has been present.

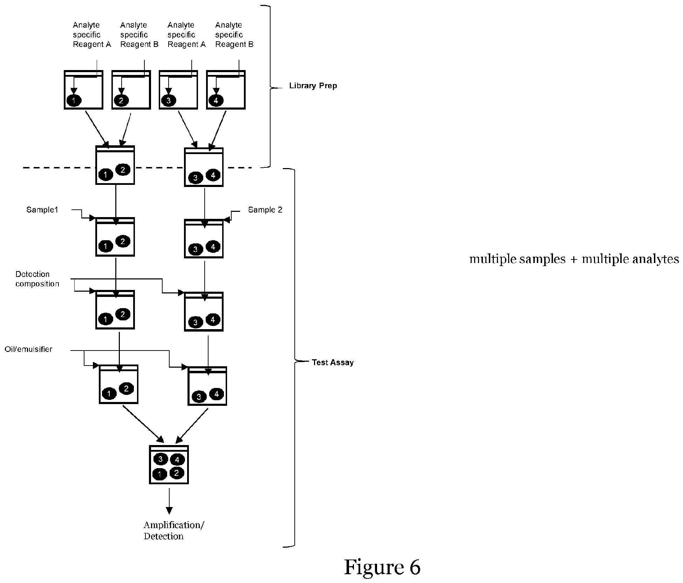

**Figure 6** shows an embodiment of a method according to the present invention in which multiple samples are tested for multiple analytes. In principle, the scheme is similar to what is shown in figure 5 except for that there will be used as many subsets of microparticles as there are samples to be tested, multiplied by the number of different analytes to be detected. As an example, the first sample in figure 6 is tested/analyzed using two different subsets of microparticles which not only differ in terms of the label component associated therewith but also in terms of the analyte-specific reagent attached to such type. The same is done for the second sample ("sample 2"). Hence, in this example there are altogether 4 different subsets of microparticles, with each of them having its unique combination of label component and analyte-specific reagent. Thereafter, the respective necessary (generic) detection reagent/detection composition is added, but still on a per-sample-basis, in order to avoid a cross-contamination between different samples. Thereafter, the respective microparticles are transferred, again, on a per-sample-basis into the non-aqueous phase, and only after these respective microparticles have been insulated in such a manner and can no longer interfere with and cross contaminate each other, the necessary detection reaction is performed. The final result will be a finding whether or not each sample tested had none, one or two of the analytes to be detected.

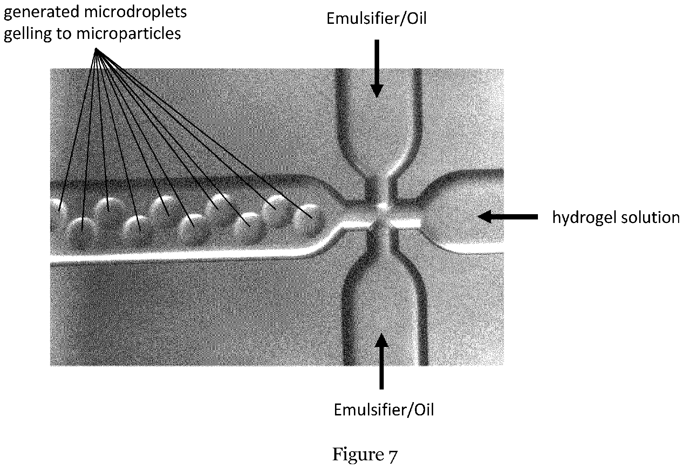

**Figure 7** shows a micrograph of the microfluidic cross junction of the set up used to generate microparticles according to the invention. Microdroplets are formed in oil and form an aqueous hydrogel solution (at the left). Subsequently the droplets are cooled and microparticles formed.

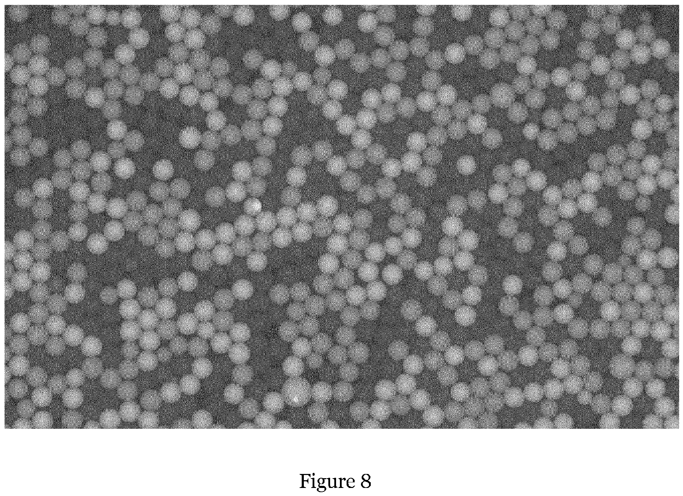

**Figure 8** shows a fluorescence micrograph acquired with a Cy3 Filter Set on a Zeiss Axioscope equipped with a digital camera. Three different fluorescence levels can be seen (bright, medium, low) representing three concentrations of the label component (Cy3 dye), corresponding effectively to three different label components.

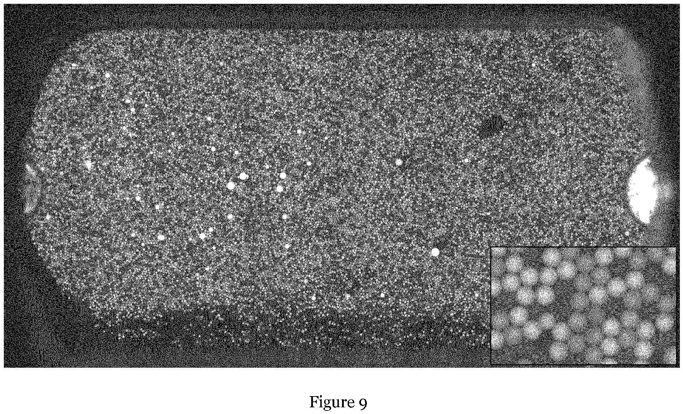

**Figure 9** shows a stitched image of the detection chamber representing the fluorescence channel encoding the label of the beads. Three different types of microparticles (or "beads"), distinguishable (and thus encoded) by their respective label components, can be clearly recognized (bright, medium and low signal) in the enlarged image insert on the lower right. Because of their different label components, these beads can be used to label or "encode" different samples (and are therefore also sometimes herein referred to as "sample encoding beads"). The beads are spatially distributed in hexagonal close packing. The total number of all Sample encoding beads (mean diameter d=105µm) recognized by the employed software algorithm within this PCR chamber has been determined with N=17.698.

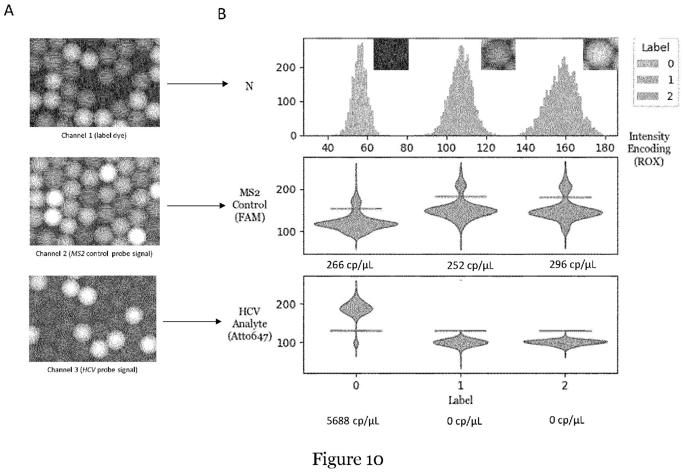

**Figure 10** **A** shows fluorescence images of color-labeled agarose-gelatin hybrid microparticles post PCR amplification. Three labels can be recognized by their fluorescence intensity in channel 1. Channel 2 represents fluorescence signals for the internal process control (MS2 phage) and channel 3 represents fluorescence specific for PCR amplification of the HCV target. Each label corresponds to a different sample that has been processed and analyzed. **Figure 10B** (upper graph) shows the histograms for the determined fluorescence intensity for the different beads from channel 1 (upper image in Fig 10A). Three separate label species are found. Violin plots representing the signal distribution found across the respective bead species for channel 2 (MS2) and channels (3) are shown in the middle and lower graphs. By applying Poisson analysis the found numbers are translated into a specific copy number per volume sample applied to the beads.

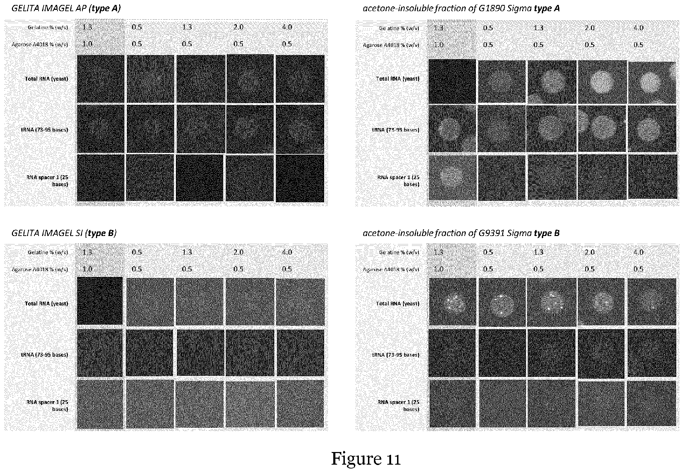

**Figure 11** summarizes the data from example 3 on the binding efficiency of different microparticle formulation for RNA. The data shows that adjustments to the composition of the microparticles allow for optimization of binding characteristics.

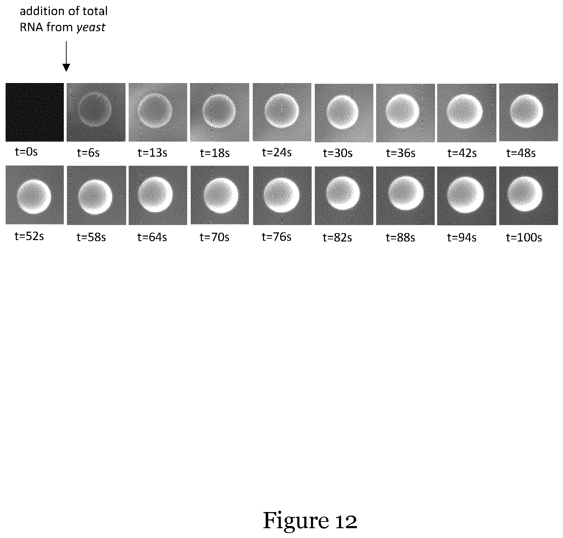

**Figure 12** shows a time course fluorescence images showing RNA binding to the prepared microparticles in Example 3.

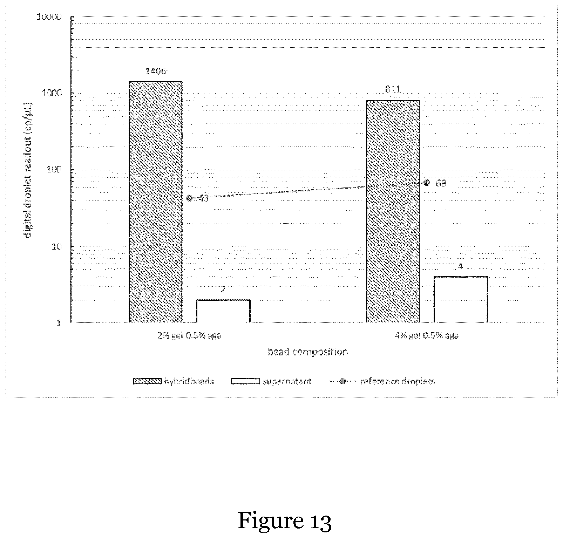

**Figure 13** illustrates the enrichment effect achieved by the use of the microparticles. The graph shows data for two different compositions of microparticles. Black dots indicate the target concentration in the sample before incubation with the microparticles determined by digital RT-PCR. White columns represent the target concentration detected after the incubation in the supernatant, grey columns target concentration on the microparticles. The enrichment effect is clearly visible, showing a depletion of the respective supernatant(s).
**Figure 14** shows the precision of the combined digital and real-time quantitative PCR analysis approach for target quantitation in microparticles. Confidence intervals are shown for both methods. The vertical line at λ=5 cp/microparticle (cp=copies) indicates the approximate value at which methods can be used interchangeably ("CI"= confidence interval).
**Figure 15** shows real time fluorescence data obtained from an image series collected on microparticles in oil during PCR amplification. A stitched image of a detection chamber with the endpoint fluorescence signal detected in one fluorescence channel specific for amplification is shown on the left. The graph in the center shows exemplarily fluorescence intensity for 12 representative individual microparticles selected from the fluorescence image on the left over time. A distribution of the calculated ct values for each microparticle detected in the fluorescence image is shown in the histogram on the right.

Moreover, reference is made to the specific description, in particular the following examples, which are given to illustrate not to limit the present invention.

### Examples

### Example 1: Generation of "Sample Encoding Beads" for a RNA detection assay

Differently labeled Microparticles with a matrix comprised of Gelatin and Agarose have been generated, whereby the Gelatin fraction of the composition served as the dye carrying part of the matrix and because of its composition provided for target binding and enrichment.

### The labeling of gelatin with fluorescent dyes for creating a particle code was carried out as following:

The acetone insoluble fraction of gelatin from bovine skin type A is labelled with a fluorescent dye taking advantage of NHS coupling chemistry. Cy®3 Mono NHS Ester (GE Healthcare) were dissolved in DMF to make a final solution of 10% (w/v). Component 1 is dissolved in 70mM sodium phosphate buffer (pH 8.0-8.3, sterile filtered) to make a final concentration of 0.25% (w/v). A 10-fold lower molecular amount of the respective dye over free gelatin amino groups is utilized to label 25mL of either gelatine type. The label solutions are incubated at 4°C overnight using the Multi-Rotator PTR-60 (Grant-bio) in the vertical mode. Fluorescently labelled gelatin purification and its concentration is accomplished by repeated ammonium sulfate precipitation using a saturated (NH₄)₂SO₄ solution. Alternatively, gelatin can be in a gelled particle-sized format for coupling and can be purified without ammonium sulfate precipitation by simply washing and centrifuging at ambient temperature. Also, ultrafiltration, solvent extraction with isopropanol, acetone or methanol, gel filtration using sepharose columns or dialysis can be performed to purify the gelatin.

In either case, purification is repeated until effluent appears clear and shows no fluorescence. Purified fluorescently labelled gelatin samples are finally vacuum-dried after washing the pellet four times with dH2O resulting in component 3.

### Preparation of gelatin/agarose hybrid solution:

A hybrid hydrogel solution consisting of three components is prepared for fabricating nano-reactors.
*Component 1*: Acetone-insoluble gelatin from bovine skin type A G1890 (Sigma)
*Component 2:* Low-gelling 2-Hydroxyethyl agarose (A4018, Sigma)
*Component:* Cy3-labeled gelatin (type A)

To generate a homogeneous 4% (w/v) solution of component 1, 40mg of component 1 is dissolved in 1mL nuclease-free water (Carl Roth) and incubated at 50°C under gentle agitation (750rpm). Likewise, 20mg of component 2 is dissolved and melted in 1mL nuclease-free water and incubated at 80°C under gentle agitation to prepare a homogeneous 2% (w/v) agarose solution. To prepare a 4% (w/v) solutions of labelled gelatin, a dried pellet of component 3 is taken up in a respective volume of nuclease-free water and incubated at 55°C until the gelatin is molten. All three components are mixed and filled up with nuclease-free water to generate a hybrid hydrogel solution with final concentrations of 1.5% (w/v) for gelatin and 0.5% (w/v) for agarose A4018, respectively. Various volumes of component 3 and component 1 are mixed yielding n distinctly colored microparticle sets. In this embodiment resuspended component 3 and component 1 are mixed in ratios 1:1000, 1:500, 1:250 to accomplish three individual label components to allow for identification and analysis of three different samples. All solutions were kept at 55°C until further use.

### Generation of un-crosslinked gelatin/agarose microparticles

Monodisperse color coded agarose-gelatin hybrid microparticles were fabricated using a microfluidic particle generator system (Dolomite, UK). Monodisperse hybrid microparticles are fabricated in a one-step process of emulsion formation using a simple flow-focus device. In detail, a standard droplet junction chip (100µm) of fluorophilic nature is used with a 4-way linear connector and a chip interface H to interface the fluidic connection between tubing and chip. Two Mitos P-Pumps deliver the hydrogel solution and the carrier oil. The system is modified with the integration of a heating rig which is placed on top of a hot plate and allows for maintaining the gelatin/agarose hybrid solution in liquid state and heating up the driving fluid ensuring consistent temperature when oil and gelatin/agarose hybrid solution contact at the chip junction. Picosurf 2 (Spherefluidics, UK) and the hybrid hydrogel solution are both prefiltered with a 0.22µm filter before placing them into the P-Pump (Mitos) and the hydrogel reservoir within the heating rig of the droplet system, respectively. Temperature of the heating rig is set to 55°C. The fluid lines are primed at 2000mbar for 1 min using the Flow Control Software. A flow rate of 15-20µl/min is adjusted for stable droplet formation. Parameters are monitored with the Dolomite Flow Control Advanced Software.
**Figure 7** shows a micrograph of the microfluidic cross junction with microdroplets forming on the left in the oil phase. Subsequently the hydrogel droplets solidify ad become microparticles.
In both cases, the color-coded agarose-gelatin hybrid microparticles are collected on ice in either 2mL microcentrifuge tubes or 15mL falcon tubes to initiate solidification of the hybrid hydrogel. To prevent loss of aqueous phase of the microparticles at the oil-air boundary, 500µL of the emulsion oil containing the microparticles is overlaid with 500µL of nuclease-free water. Microparticles are subsequently cooled down to 4°C for at least 24 hours (preferentially 48h) to form stable hybrid scaffolds.

### Recovery of hybrid microparticles from continuous phase

Solidified hybrid beads accumulate on top of the emulsion oil. The emulsion oil is removed carefully with a pipette taking care not to remove the particles. Afterwards, 500µL iH,iH,2H,2H-perfluorooctanol (PFO; Sigma) is added to the tube to break the emulsion. To transfer the hybrid hydrogel particles into the oil phase, the tube is vortexed for 5s and centrifuged at 2,500 x g for 5s. The hybrid hydrogel microparticles are transferred to a fresh 1.5 mL microcentrifuge tube.
Optional: This procedure can be repeated to remove residual fluorocarbon oil and surfactant. Following the PFO wash, recovered microparticles are washed twice with 1mL nuclease-free water. Microparticle quality and sizes are visually examined using a microscope. The final result of the described procedure yields nanoreactor microparticles (diameter d=100µm) sets with a porous polymeric matrix and a sample encoding capacity.
**Figure 8** shows a fluorescence micrograph acquired with a Cy3 Filter Set on a Zeiss Axioscope equipped with a digital camera. Three different fluorescence levels can be seen (bright, medium, low) representing three concentrations of the dye (Cy3 dye), and thus effectively three different label components allowing a distinction between the respective microparticles labelled therewith.

### Lyophilisation of microparticle sets

Optionally, the microsphere sets can be lyophilized to give microparticle pellets for long-term storage. Therefore, desired microsphere sets, and concentrations are supplemented with an equal volume of a 600mg/mL trehalose solution to generate a 30% (w/v) trehalose containing microparticle library slurry. Subsequently, library aliquots of 100µl are prepared in RNase/DNase-free PCR strip tubes ready for lyophilisation. The type of excipient (e.g. trehalose) and its concentration in the lyophilisation formulation affects the degree of swelling of the freeze-dried microspheres when exposed to an eluate later in the process. The microsphere sets were freeze-dried under vacuum (-25°C and 0.1mbar) using the Alpha 2-4 LSCplus freeze-drier (Christ) after freezing on dry ice for 2h. The samples were left in the freeze dryer for a total time of 200min. The main drying stage was held at 0.01mbar for 3h with a stepwise increase in temperature, from -25°C to 25°C. The final drying step was conducted at 25°C and 0.05mbar for 20min.

### Example 2: Encoding of samples with "Sample Encoding Beads"

### Sample Preparation using Hybrid beads

### Preparation of Gelfiltration Resin

Dry P-2 Bio-Gel Media Extra fine <45 µm (BIO-RAD 150-4118) is allowed to hydrate in nuclease-free deionized H₂O. Subsequently the gel is filled into an empty spin column (i.e. SigmaPrep™ spin columns, Sigma-Aldrich SC1000-KT) to yield a final bed volume of 400 µl. The column is subjected to a short centrifugation step of 1 min at 1000 rcf to remove the mobile phase. The column is washed two times with 400 µl of 50 mM Tris HCl buffer pH 8.4 by adding the buffer and subsequently spin the columns as described above.

### Sample Lysis

Three Samples of 30 µl of whole blood obtained from a healthy donor (Institute of Transfusion Medicine, University Hospital Jena) have been spiked with 300cp/µL of MS2 virus (Sample 1, 2 and 3) and one of the three samples (Sample 1) has been spiked with AccuPlex HCV Recombinant Sindbis Virus (SeraCare 0505-0036) diluted in Basematrix Negative Diluent (SeraCare 1805-0075), resulting in a nominal titer of 5.500cp/µL sample.

All sample have been separately mixed with 195 µl of lysis buffer each, containing 4.5 M Guanidinium HCl, 9% Triton X100, 18 mM EDTA and 100 mM Tris HCl pH 8.0. Each lysis mix has incubated at 65°C for 5 minutes.

### Gelfiltration to remove Guanidinium HCl

Immediately after lysis each mix has been applied to the P-2 column. The flow through/filtrat has been recovered after 1 minute of centrifuation at 1.000 rcf into a fresh reaction tube containing 200U of RNase Inhibitor (biotechrabbit, DE).

### Binding of the analyte to Sample Encoding Beads

Samples are assigned to the fluorescently labelled microparticles as shown in the following Table.

**Assignment of samples to Sample Encoding Beads**

| | Sample Encoding Subtype | *HCV* | *MS2* |
|---|---|---|---|
| Sample 1 | 0 | + | + |
| Sample 2 | 1 | - | + |
| Sample 3 | 2 | - | + |

| | | | |
|---|---|---|---|
| + = positive; -= negative | | | |

Pellets of lyophilized microparticles are resuspended in 70µL RNA-containing binding buffer (component 4), with each set consisting of roughly 10.000 nanoreactors available for digital PCR analysis. These are allowed to absorb the buffer and to instantly swell providing for a functional porous 3D gelatin-agarose matrix developed for efficient and unspecific binding of nucleic acids and digital PCR compatibility. Individual samples are incubated with their corresponding beads at 15°C for 5min at 1000rpm in separate tubes to fully capture HCV and MS2 RNA resulting in an enrichment of nucleic acids in the nanoreactors.

### Washing the microparticles

200 µl of washing buffer (50 mM Tris HCl pH 8.4, 1 U/µl RNaseInhibitor) were added to each sample. After a short vortexing step at 12.000 to 16.000 rpm for 1 second, the microparticles are sedimented by centrifugation at 300 rcf for 30 seconds and the supernatant is removed. The washing step is repeated 3x.

### 1. Detection and Quantitation of a molecular target in different samples with "Sample Encoding Beads"

### Loading the reagents onto the microparticles

The reagents for the detection of an analyte within the nano-reactors are supplied as a freeze-dried pellet that is resuspended with 100µL of 1x PCR buffer (component 7) making a 2x reagent mixture (component 8). The reagents are carefully resuspended by a short vortexing step. An equal volume of the reagent mix with regard to the bead slurry is distributed into the individual containers with individual sample encoding bead sets that have captured the RNAs. The amplification and detection reagents are allowed to diffuse into (and bind to) the hydrogel matrix by a short incubation of 5 min at 15°C while shaking at 1000rpm.
*Component 7*: PCR buffer (20mM Tris HCl, 22mM KCl, 22mM NH4Cl, 3mM MgCl2, pH 8.5)
*Component 8:* Reagent mixture (2x)
   - 2x thermostable Reverse Transcriptase with Ribonuclease Inhibitor (biotechrabbit GmbH)
   - 0.4 U/µl Hot Start *Taq* DNA Polymerase (biotechrabbit GmbH)
   - 0.8 mM dNTPs (biotechrabbit GmbH)
   - 0.2% (w/v) low bioburden, protease free, for molecular biology BSA (Sigma)

### Analyte-specific reagents (HCV)

- 0.8µM *HCV*-Brun sense primer (5'-GTGGTCTGCGGAACCGGTGA-3') SEQ ID NO:1
- 0.8µM *HCV*-Brun antisense primer (5'-CGCAAGCACCCTATCAGGCAGT-3') SEQ ID NO:2
- 0.8µM *HCV*-Brun TaqMan probe (5'-Att0647-CCGAGTAGYGTTGGGTYGCGAAAGG-BHQ-2-3') SEQ ID NO:3

### Internal Process Control-specific reagents (MS2)

- 0.8µM *MS2*-Nino sense Primer (5'-CTCTGAGAGCGGCTCTATTGGT-3') SEQ ID NO:4
- 0.8µM *MS2*-Nino sense Primer (5'-GGTCCCTACAACGAGCCTAAATTC-3') SEQ ID NO:5
- 0.8µM *MS2*-Nino TaqMan probe (5'-FAM-TCAGACACGCGGTCCGCTATAACGA-BHQ-1-3') SEQ ID NO:6

### Emulsification of Microparticles

Individual nano-reactor sets are transferred separately into a non-aqueous phase by dispersing microparticles in component 9 to prevent crosstalk between the sample-encoded beads. The beads are centrifuged at 500rcf for 30s and the supernatant is discarded. The bead bed is brought in contact with an excess of component 9 (200µL) using a 1.5mL microcentrifuge tube. High shear forces need to be applied to deagglomerate and emulsify aqueous microparticles in the fluorocarbon oil into single nano-reactors. The mixtures are agitated by either application of ultrasound using the Sonifier™ S-450 and the Ultrasonics Sonifier™ Cup Horn (Branson) or by simply sliding the tube over the holes of a microcentrifuge tube rack 20 times at a frequency of approximately 20/s while pressing the tube against the rack surface. This applies mechanical stress and breaks attracting forces between the aqueous microparticles and creates surface tension forming a suspension/emulsion. Both the hydrogel microparticles and excess aqueous phase are emulsified in the oil phase. The submicron-scaled droplets that are produced as a byproduct are eliminated by washing the emulsion three times by mild centrifugation (500rcf). Repeated washing with the same oil (component 12) removes essentially all undesirable liquid droplets. Component 12 also yields efficient thermostability of the emulsion for subsequent digital emulsion PCR. The three emulsified Sample Encoding Bead sets can now be simply combined into one container (tube) prepared for parallel digital signal amplification and detection of encoded samples.

### Component 9: phase transfer & signal amplification oil

- HFE-7500 fluorocarbon oil (3M Deutschland GmbH) supplemented with
- 2-5% (v/v) PicoSurf (Dolomite Microfluidics) OR 2-5% (v/v) FluoSurf (Emulseo) OR 2-5% (v/v) 008-FluoroSurfactant (RAN Biotechnologies)

### Parallel digital PCR amplification reactions in Sample Encoding Beads

The monodispersed emulsion with encapsulated sample 1, sample 2 and sample 3 is transferred into a detection chamber with an area of approximately 2.5 cm² and a layer thickness of 100 µm. The chamber detection window is made of a 0.8mm Polycarbonate (Makrolon 6555; Covestro AG) while the opposite side of the chamber is composed of polished and unmodified transparent 125-micron Polycarbonate (Lexan 8010) film (Koenig Kunststoffe GmbH) facilitating efficient heat transfer necessary for individual nanoliter reactions. Nanoreactors suspended in the fluorocarbon oil are forced to form a monolayer owing to the dimension of the reaction chamber. Thus, microspheres provide an evenly spaced array of approximately 20.000-30.000 nano-reactors (≈7.000-10.000/sample) for subsequent parallel signal amplification reactions.

Microparticles are subjected to ultra-rapid temperature cycling using a modified PELTIER element 30x30x4.7mm, 19.3W (Quick-Ohm, Küpper & Co. GmbH, #QC-71-1.4-3.7M) and an established chamber-specific PCR control mode. The thermal RT-PCR conditions applied are: Reverse Transcription for at 50°C for 10min, Initial denaturation at 95°C for 30s followed by 30-45 cycles of a two-step PCR consisting of Denaturation at 95°C for 2s and Annealing/Elongation at 65°C for 5s. Due to their sol-gel switching capability, the suspension becomes an emulsion with individual liquid nanoliter droplets. The multiple duplexed amplifications of HCV and MS2 (control) from different samples takes place in the labelled nano reaction compartments.

Automated Image acquisition is triggered by the BLINK toolbox software and is done with a Fluorescence microscope (Zeiss AxioObserver) equipped with a 5x objective (field of view 4.416mm x 2.774mm) and a pE-4000 (CoolLED Ltd.) light source. The microscope was further equipped with three fluorescence filter sets (Cy5 ET, Cy3 ET, FITC/FAM HC, AHF Analysentechnik) and an automated x-y stage to which the thermocycler with the reaction chamber was mounted.

Image acquisition settings are as following: 100-1000ms and gain of 1-10x. One image is required for label identification (λexc 1 = 580nm), one image for the internal control PCR signals (Xexc 2 = 470nm) and one image for specific PCR signals (λexc 3 = 635nm). For optional nanoreactor specific real-time analysis, three images corresponding to three fluorescence channels can be taken at each cycle at a suitable position of the chamber.

Upon completion of the thermal protocol, the whole detection chamber field is scanned using the same equipment at the settings mentioned above. In total 48-56 images are required to cover the dimension of the amplification/detection chamber.

A stitched image of the detection chamber representing the fluorescence channel encoding the label of the beads is shown in **Figure 9****.** Three different Sample Encoding Beads can be clearly recognized (bright, medium and low signal) in the enlarged image insert on the lower right. The beads are spatially distributed in hexagonal close packing. The total number of all Sample encoding beads (mean diameter d=105µm) recognized by the employed software algorithm within this PCR chamber has been determined with N=17.698.

### Decoding of samples and endpoint analysis

All images acquired are subjected to an automated multifaceted image processing algorithm. The method employs image segmentation exploiting the Maximally Stable Extremal Regions (MSER) to detect neighboring droplets from the result of MSER-based image segmentation. In detail, images are first subjected to preprocessing involving a median filter. Secondly, the MSER algorithm is applied to the image background to determined convex turning points of background outlines and their Delaunay triangulation to identify appropriate cuts between the droplets/microparticles. Furthermore, droplets/microparticles are segmented using the MSER algorithm. Finally, a plausibility check for droplet/microparticle outlines is performed (contrast, form, convexity). Features including fluorescence signals in each channel, position, diameter/volume, etc. of all segmented droplets/microparticles are subsequently collected. Experiment data is applied to a Jupyter script identifying individual labels (graduation of coupled Cy3 dye) and their respective specific amplification.

The PCR is amplified to an endpoint and thus the total number of fluorescent positive and negative droplets is determined for each individual label. Positive droplets contain at least 1 copy of the specific target and thus show an increase fluorescence signal above a defined intensity threshold. The threshold value is derived from previously performed amplification reactions without template or is determined in each experiment using statistics. Negative and positive fractions for each nanoreactor type are clustered and the fraction of positive droplets is fitted to a Poisson algorithm to determine the initial concentration of the target RNA molecules in units of copies/µL (copies/mL) input. Since the assay described above provides simultaneous detection of HCV in 3 different samples, the reactors cluster into 6 groups:

**Digital PCR endpoint data cluster.**

| Cluster | Sample | Sample Encoding Bead type (label) | *HCV* RNA | *MS2 phage* RNA |
|---|---|---|---|---|
| 1 | Sample 1 | 0 | Positive | Pass |
| 2 | | | Negative | |
| 3 | Sample 2 | 1 | Positive | Pass |
| 4 | | | Negative | |
| 5 | Sample 3 | 2 | Positive | Pass |
| 6 | | | Negative | |

Fluorescence images of color-labeled agarose-gelatin hybrid microparticles are shown in **Figure 10A** post PCR amplification. Three labels can be recognized by the their fluorescence intensity in channel 1. Channel 2 represents fluorescence signals for the internal process control (MS2 phage) and channel 3 represents fluorescence specific for PCR amplification of the HCV target. Each label corresponds to a different sample that has been processed and analyzed. Figure 10B (upper graph) shows the histograms for the determined fluorescence intensity for the different beads. Three separate label species are found. Violin plots representing the signal distribution found across the respective bead species for channel 2 (MS2) and channels (3) are shown in the middle and lower graphs. By applying Poisson analysis the found numbers are translated into a specific copy number per volume sample (cp/µl) applied to the beads.

### Example 3: RNA binding capability of agarose-gelatin hybridbeads

Binding of RNA is examined for various hybrid beads consisting of various concentrations of different gelatins and agarose A4018. Bound RNA on microparticles was either measured qualitatively by using an RNA dye assay or quantitatively using the digital PCR format described above.

### Preparation of beads

Microparticles were generated as described before with the following deviations:
- Labelled fraction of gelatin was omitted thereby producing unlabelled microparticles
- The hybrid hydrogel solutions for generation of microparticles consisted of different concentrations of agarose and gelatin, namely:
   a) 1.3% gelatin, 1.0% agarose
   b) 0.5% gelatin, 0.5% agarose
   c) 1.3% gelatin, 0.5% agarose
   d) 2.0% gelatin, 0.5% agarose
   e) 4.0% gelatin, 0.5% agarose
- Two different gelatin types each of two producers were used for microparticle fabrication, namely:
   ∘ GELITA Imagel AP (typeA)
   ∘ GELITA Imagel SI (typeB)
   ∘ acetone-insoluble fraction of G1890 (Sigma, type A)
   ∘ acetone-insoluble fraction of G9391 (Sigma type B)

### RiboGreen RNA binding assay

Wells of UV-Star microtiter plates (Greiner) are filled with 3µL of a 50% bead slurry in a 1x binding buffer (component 4). Final concentrations of 1 ng/µL of RNA from different preparations, namely an RNA-spacer (Metabion), a tRNA from brewer's yeast (Roche Diagnostic) and a total RNA extracted from blood are incubated at 15°C for 5-10min allowing the RNA to penetrate into/onto the porous hydrogel matrix and bind. As binding occurs quickly, mixing directly after addition of RNA to the beads is crucial for homogenous distribution of RNA across the beads.
To visualize RNA that is bound to the hybrid microparticles 0.5µL of a 1:2 diluted Quant-iT™ RiboGreen® RNA reagent aliquot is pipetted to the microtiter wells and mixed. The beads were washed twice with 200µL of TE buffer and their fluorescence signal subsequently measured using a Fluorescence microscope (Zeiss AxioObserver) and a pE-4000 (CoolLED Ltd.) light source.

**Figure 11** illustrates that adjustments to the composition of the microparticles allow for optimization of binding characteristics. GELITA Imagel A gelatine in combination with A4018 leads to relatively low RiboGreen signals on the beads in comparison to the aceton-insoluble fraction of G1890 type A gelatine from Sigma. Total RNA, tRNA and the short RNA fragment can be bound to a greater extent with hybrid beads made of >1.3% gelatine in combination with 0.5% agarose. In combination with 1% agarose, better signals are also obtained for the RNAs tested (total RNA sample results are missing). Gelatine type B behaves differently in comparison to type A with regard to RNA enrichment on the beads. While almost no signals are visible for any of the GELITA SI gelatine beads for any of the RNAs (meaning only diffusion into particle but no enrichment), the beads made of the acetone-insoluble fraction of G9391 gelatine typeB show RiboGreen signals although those are weak for RNA spacer 1 and tRNA. Overall total RNA, tRNA and the short RNA fragment can be bound to a greater extent with hybridbeads made of >1.3% gelatine in combination with 0.5% agarose.
**Figure 12** shows binding of total RNA from yeast over the time to microparticles visualized with Quant-iT™ RiboGreen® RNA reagent. The assay format also allows for direct monitoring of RNA binding to the microparticles. Images have been acquired every 6s and the increase in RiboGreen signal can be directly measured.

### Quantifying RNA binding by digital PCR

For quantitative assessment of RNA binding capacity of the microparticles according to the invention digital PCR within the nanoreactors was performed as described above. The ability of the microparticles to transition from a solidified particle to a liquid droplet allows for providing both the binding and digital PCR detection matrix. Thus, bound *HCV* RNA molecules can be measured directly on the beads. Alternatively, the supernatant can be measured in a digital format using the DG8 Cartridge (Bio-Rad) for droplet generation.
40µL of binding buffer (component 4) containing purified (QIAamp Viral RNA Mini Kit) Accuplex HCV RNA (Seracare Life Sciences Inc.) were added to 40µL of a bead bed and incubated at 15°C for 5min at 1000rpm to bind the RNA. Beads were subsequently centrifuged at 300xg for 30s and the supernatant is kept on ice for subsequent analysis. 40µL of the 2x reagent mixture (component X) is added to the bead bed (40µL) and the amplification and detection reagents diffuse into the hydrogel matrix by a short incubation of 5 min at 15°C while shaking at 1000rpm.

### Component X: Reagent mixture (2x)

- 2x thermostable Reverse Transcriptase with Ribonuclease Inhibitor (biotechrabbit GmbH)
- 0.4 U/µl Hot Start Taq DNA Polymerase (biotechrabbit GmbH)
- 0.8 mM dNTPs (biotechrabbit GmbH)
- 0.2% (w/v) low bioburden, protease free, for molecular biology BSA (Sigma) Analyte-specific reagents (HCV)
- 0.8µM HCV-Brun sense primer (5'-GTGGTCTGCGGAACCGGTGA-3') SEQ ID NO:1
- 0.8µM HCV-Brun antisense primer (5'-CGCAAGCACCCTATCAGGCAGT-3') SEQ ID NO:2
- 0.8µM HCV-Brun TaqMan probe (5'-Att0647-CCGAGTAGYGTTGGGTYGCGAAAGG-BHQ-2-3') SEQ ID NO:3

Bead phase transfer, amplification reaction and analysis are conducted as described before. The supernatant (40µL) is supplemented with PCR reagents (40µL of 2x reagent mix) and subsequently emulsified using the DG8 Cartridge (Bio-Rad). 100µL of the emulsion reagent HFE-7500 containing 2-5% Picosurf 2 (Sphere Fluidics) and 40µL is applied into the bottom wells of the cartridge. Vacuum is applied in the collection well by pulling gently on a syringe connected to the well. Droplets are collected and transferred to a separate detection chamber for digital analysis. The settings for the amplification reaction and analysis of the droplets are identical to the settings for the beads. Also, a reference sample consisting of equal volumes of 2x reagent mix and the PCR buffer is emulsified and analysed likewise.

**Figure 13** shows summary data on these experiments that were designed to assess the enrichment capacity of two different compositions of microparticles. Black dots indicate the target concentration in the sample before incubation with the microparticles. White columns represent the target concentration detected after the incubation in the supernatant, grey columns target concentration on the microparticles. The enrichment effect is clearly visible.

**Figure 14** shows the precision of the combined digital and real-time quantitative PCR analysis approach, outlined further above, for target quantitation in microparticles. Confidence intervals are shown for both methods. The vertical line at λ=5 cp/microparticle (cp=copies) indicates the approximate value at which methods can be used interchangeably ("CI"= confidence interval).

**Figure 15** shows real time fluorescence data obtained from an image series collected on microparticles in oil during PCR amplification. A stitched image of a detection chamber with the endpoint fluorescence signal detected in one fluorescence channel specific for amplification is shown on the left. The graph in the center shows exemplarily fluorescence intensity for 12 representative individual microparticles selected from the fluorescence image on the left over time. A distribution of the calculated ct values for each microparticle detected in the fluorescence image is shown in the histogram on the right.

## Claims

1. A method of detecting and/or quantitating an analyte of interest in a plurality of biological liquid samples, said method comprising a sample-specific process part, followed by a generic process part;
said sample-specific process part comprising the following steps:
- separately providing, in any order, a plurality of different biological liquid samples suspected of containing an analyte of interest, and a plurality of differently labelled subsets of porous microparticles, wherein, in said plurality of subsets, each of said subsets is separate from the other subsets;
- specifically labelling each of said different biological samples by separately exposing each of said separate subsets of microparticles to one biological liquid sample each, thus allowing each sample to be taken up by one specifically labelled subset of porous microparticles in an aqueous environment;
- separately transferring each subset of porous microparticles from said aqueous environment to a non-aqueous environment;
said generic process part comprising the steps:
- mixing together the differently labelled subsets of porous microparticles in said non-aqueous environment and thus generating a suspension of a plurality of differently labelled subsets of porous microparticles therein;
- performing a detection reaction for detecting said analyte of interest, on said suspension of said plurality of differently labelled subsets of porous microparticles; and
- detecting and/or quantitating said analyte of interest, if present, in any of said differently labelled subsets of suspended microparticles.

2. The method according to claim 1, wherein, in said step of separately exposing each of said separate subsets of microparticles to one biological liquid sample each, each of said separate subsets of microparticles is exposed to, in any order, one biological sample each and a detection composition for performing a chemical or biochemical detection reaction, such that each subset of microparticles takes up the respective biological sample and the detection composition to which it has been exposed.

3. A method of detecting and/or quantitating an analyte of interest in a plurality of biological liquid samples, in particular according to any of claims 1 and 2, said method comprising the following steps:
a. a step of separately providing, in any order, a plurality of separate biological liquid samples suspected of containing an analyte of interest, and a plurality of porous microparticles; each of said porous microparticles having a porous matrix and being configured to receive a volume of liquid in said porous matrix; wherein, in said plurality of porous microparticles, there are different subsets of microparticles provided, each subset of microparticles being **characterized by** a specific label component that is attached to, contained in or otherwise associated with the respective subset; wherein in said step of providing, the number of different subsets of microparticles provided is at least as big as the number of separate biological liquid samples provided, and wherein furthermore in said step of providing, the different subsets of microparticles are provided separate from each other; wherein, optionally, said different separate subsets of microparticles contain in their respective porous matrix a detection composition comprising reagents for performing a chemical or biochemical detection reaction of an analyte;
b. a step of exposing each separate subset of microparticles to exactly one separate biological liquid sample, thereby allowing each separate subset of microparticles to incubate with a volume of exactly one separate biological liquid sample and to take up such sample or a portion thereof, and, optionally, to accumulate analyte, if present in said sample, in or on the matrix of the microparticle(s); and, further optionally, in case that said different separate subsets of microparticles, when provided in step a), do not yet comprise reagents for performing a chemical or biochemical detection reaction of an analyte, a step of exposing each separate subset of microparticles to a detection composition comprising reagents for performing a chemical or biochemical detection reaction of an analyte, thereby allowing each separate subset of microparticles to receive said reagents;
c. transferring each subset of microparticles separately into a non-aqueous phase and removing some or all of the aqueous phase surrounding the individual prefabricated microparticle(s) of said subset(s), thereby creating a plurality of separate subsets of insulated reaction spaces for detecting said analyte, which reaction spaces comprise an aqueous phase including sample and said reagents for performing a chemical or biochemical detection reaction of an analyte, and which reaction spaces are confined to said void volume(s) of said microparticles;
d. mixing the separate different subsets of microparticles in said non-aqueous phase, such that all of said different subsets of microparticles form a suspension of different microparticles in said non-aqueous phase; subjecting said mixed different subsets of microparticles to conditions required for performing a chemical or biochemical detection reaction of an analyte; performing such detection reaction of said analyte of interest; and detecting and/or quantitating said analyte of interest, if present, in any of said different subsets of microparticles, by means of a signal generated in said detection reaction in a respective subset of microparticles, if said analyte of interest is present in said respective subset.

4. The method according to claim 3, said method further comprising the step
e) determining which sample(s) of said plurality of samples provided in step a) does contain said analyte of interest by determining the identity of the subset(s) of microparticles in which said analyte of interest is detected in step d), wherein, preferably the identity of the subset(s) of microparticles in step e) is determined by means of the specific label component that is attached to, contained in or otherwise associated with the respective subset of microparticle(s).

5. The method according to any of claims 3 - 4, wherein step b) further comprises a substep of generating a first record of correlation indicating which separate subset of microparticles is or has been exposed to which sample, and step d) comprises a substep of generating a second record of correlation indicating in which subset of microparticles a signal has been generated in said detection reaction.

6. The method according to claims 4 - 5, wherein step e) is performed by reference to said first and second records of correlation and by linking said records, thus allowing to determine which sample(s) of said plurality of samples provided in step a) does(do) contain said analyte of interest.

7. The method according to any of the foregoing claims, wherein each of said porous microparticles has a porous matrix which allows to accumulate analyte of interest by binding to analyte through:
(i) a polymer or polymer mixture that forms or is said porous matrix; or
(ii) at least one ionisable group, or a plurality of ionisable groups, immobilized on said porous matrix, said ionisable group(s) being capable of changing its(their) charge(s) according to ambient conditions surrounding said microparticle(s); or
(iii) at least one charged group, or a plurality of charged groups immobilized on said porous matrix; or
(iv) a combination of any of (i) - (iii).

8. The method according to any of claims 1 - 6 or 7, wherein each of said porous microparticles has a porous matrix and comprises an analyte-specific reagent (ASR) that is attached to said porous matrix or contained by said microparticle, such analyte-specific reagent allowing an enrichment of an analyte of interest and/or allowing a specific signal or target amplification reaction involving said analyte; wherein said analyte-specific reagent is capable of specifically binding to an analyte of interest, wherein, preferably the analyte-specific reagent is selected from nucleic acids, including aptamers, Spiegelmers; antibodies or antibody fragments; non-antibody proteins capable of specifically binding an analyte or analyte complex, such as receptors, receptor fragments, and affinity proteins; wherein, preferably, said analyte-specific reagent is selected from nucleic acids, in particular nucleic acid oligomers and nucleic acid primers.

9. The method according to claim 8, wherein each of said porous microparticles contains or has the same analyte-specific reagent attached to its porous matrix.

10. The method according to claim 9, wherein, in said plurality of porous microparticles, there are different subsets of microparticles,
with each subset
- having its distinct label component attached to, contained in or otherwise associated with said microparticles of said subset; and
all of said different subsets having
- the same analyte-specific reagent attached to or contained in said microparticles of said subsets, said analyte-specific reagent being specific for one analyte of interest;
such that said different subsets of microparticles are identical in terms of the analyte-specific reagent attached or contained, but differ by
- the respective label component attached to, contained in or otherwise associated with said microparticles of each subset;
with each subset being unambiguously defined and identifiable by said respective label component.

11. The method according to any of claims 1 - 7, or according to any of claims 8 - 10, wherein said method is a method of detecting and/or quantitating one analyte of interest in a plurality of biological liquid samples, wherein the number of different subsets of microparticles provided, preferably provided in step a), equals the number of separate biological liquid samples provided.

12. The method according to claim 8, wherein, in said plurality of porous microparticles, there are several different analyte-specific reagents attached to or contained in said microparticles.

13. The method according to claim 12, wherein there are different subsets of microparticles,
with each subset
- having its distinct label component attached to, contained in or otherwise associated with said microparticles of said subset; and
wherein furthermore, in said plurality of porous microparticles, there are different classes of subsets of microparticles with each class of subsets
- having a different analyte-specific reagent attached to the porous matrix of said microparticles or contained in said microparticles; wherein, preferably, there are at least two different classes of subsets of microparticles, more preferably at least three or more different classes of subsets of microparticles;
such that said different subsets of microparticles differ by the respective label component attached to, contained in or otherwise associated with said microparticles of each subset; and each subset of microparticles forms part of one class of subsets of microparticles; with each subset being unambiguously defined and identifiable by the respective label component and the respective analyte-specific reagent; and
such that said different classes of subsets of microparticles differ by the respective analyte-specific reagent attached or contained; and each of said different classes comprises several subsets of microparticles, all of which subsets have the same analyte-specific reagent attached or contained.

14. The method according to any of claims 12- 13, wherein said method is a method of detecting and/or quantitating more than one analyte of interest in a plurality of biological liquid samples, wherein, the number of different subsets of microparticles provided, preferably provided in step a), equals the number of separate biological liquid samples provided, multiplied by the number of analytes of interest to be detected, and wherein there are as many classes of subsets of microparticles provided, preferably provided in step a), as the number of analytes of interest to be detected.

15. The method according to any of claims 3 - 14, wherein said porous matrix is a porous polymer matrix formed by a polymer or polymer mixture, wherein, preferably, said porous polymer matrix is composed of a polymer (or polymers) that is (are) not crosslinked, wherein, more preferably, said polymer or polymer mixture that forms said porous polymeric matrix, is composed of agarose or a combination of agarose and gelatin, wherein, more preferably, in said combination of agarose and gelatin, said agarose is present in a range of from 0.1 %(w/v) to 4%(w/v), and said gelatin is present in a range of from 0.1%(w/v) to 20%(w/v), preferably from 0.5% (w/v) to 20%(w/v).

16. The method according to any of claims 3 - 15, wherein said analyte of interest is a nucleic acid, said detection reaction is a nucleic acid amplification, and said detection composition is a composition for performing a nucleic acid amplification which comprises a buffer, mono-nucleoside-triphosphates, an amplification enzyme, such as a suitable nucleic acid polymerase, e.g. Taq polymerase, and a nucleic acid dye for the detection of an amplification product, such as an amplified nucleic acid, and, optionally, one or more pairs of amplification primers and, further optionally, respective molecular probes (e.g. TaqMan Probes, molecular beacons, etc.), if such primers and/or probes are not already provided as analyte-specific reagent(s) (ASR) being attached to or contained in said microparticles; AND/OR
wherein said analyte of interest is a protein or other non-nucleic acid molecule, said detection reaction is an immunochemistry detection reaction, and said detection composition is a composition for performing such immunochemistry detection reaction and is provided in said method as two separate components: wherein a first component of said detection composition comprises necessary reagents for performing an immunochemistry detection reaction, such as a buffer, and a secondary antibody or secondary antibody fragment coupled to a suitable reporter enzyme and being specific for the same analyte as a primary antibody, antibody fragment, or non-antibody protein, used as analyte-specific reagent (ASR) in said immunochemistry detection reaction; and, optionally, a primary antibody, antibody fragment, or a non-antibody protein capable of specifically binding said protein analyte or other non-nucleic acid analyte, if such a primary antibody, antibody fragment, or non-antibody protein is not already provided as analyte-specific reagent(s) (ASR) being attached to or contained in said microparticles; and wherein a second component of said detection composition comprises, as a detection reagent, a suitable substrate for said suitable reporter enzyme which substrate upon having been reacted by said reporter enzyme, becomes detectable, preferably optically detectable, more preferably detectable by fluorescence.

17. The method according to any of the foregoing claims, wherein, in said step of detecting and quantitating said analyte of interest, quantitation of said analyte is performed by a method selected from:
a) digital nucleic acid amplification, in particular digital polymerase chain reaction (PCR);
b) real-time quantitative nucleic acid amplification, in particular real-time polymerase chain reaction (PCR);
c) immunochemistry detection methods, in particular digital immunochemistry detection methods, such as digital immunoassays, e.g. digital enzyme-linked immunosorbent assays (ELISAs);
d) immunochemistry detection methods, in particular digital immunochemistry detection methods, combined with nucleic acid amplification, such as immuno-polymerase chain reaction; in particular digital immune-PCR; and
e) a combination of any of a) - d);
wherein quantitation is performed using any of methods a) or b), or a combination of a) and b), if the analyte of interest is a nucleic acid; and wherein quantitation is performed using any of methods c) or d), if the analyte is a protein, peptide or other non-nucleic acid analyte.

18. A kit for detecting an analyte of interest in a plurality of biological liquid samples, in particular for performing the method according to any of claims 1 - 17, said kit comprising:
- a plurality of containers comprising a plurality of microparticles, each container comprising a subset of said plurality of porous microparticles, with each of said porous microparticles within each subset having a porous matrix and being configured to receive a volume of liquid in said porous matrix; each subset of microparticles being **characterized by** a specific label component that is attached to, contained in or otherwise associated with the respective subset, wherein, preferably, said microparticles are as defined in any of claims 1 - 17; optionally, a container comprising an aqueous washing reagent for washing said microparticles;
- a container comprising a detection composition for detecting an analyte of interest; said detection composition comprising reagents for performing a chemical or biochemical detection reaction of an analyte; wherein said detection composition is either a composition for performing a nucleic acid amplification, or is a composition for performing an immunochemistry detection reaction;
- a container comprising a non-aqueous phase for transferring said different subsets of microparticles into a non-aqueous phase, once each subset has been exposed to a biological liquid sample, and for generating separate different suspensions of subsets of microparticles in a non-aqueous phase;
- a mixing container for mixing the separate different suspensions of subsets of microparticles in said non-aqueous phase together, such that all of said different suspensions of subsets of microparticles form a single suspension of different microparticles in said non-aqueous phase which is then subjected to a detection reaction;
- a container for performing a detection reaction.

19. The kit according to claim 18, wherein each of said porous microparticles has an analyte-specific reagent (ASR) attached to its porous matrix or contains an analyte-specific reagent (ASR), such analyte-specific reagent allowing an enrichment of an analyte of interest and/or allowing a specific signal or amplification reaction involving said analyte; wherein said analyte-specific reagent is capable of specifically binding to an analyte of interest, wherein, preferably, said analyte-specific reagent is selected from nucleic acids, including aptamers, Spiegelmers; antibodies or antibody fragments; non-antibody proteins capable of specifically binding an analyte or analyte complex, such as receptors, receptor fragments, and affinity proteins; wherein, preferably, said analyte-specific reagent is selected from nucleic acids, in particular nucleic acid oligomers and nucleic acid primers.

20. The kit according to any of claims 18 - 19, wherein said analyte of interest is a nucleic acid, said detection reaction is a nucleic acid amplification, and said detection composition is a composition for performing a nucleic acid amplification which comprises a buffer, mono-nucleoside-triphosphates, an amplification enzyme, such as a suitable nucleic acid polymerase, e.g. Taq polymerase, and a nucleic acid dye for the detection of an amplification product, such as an amplified nucleic acid, and, optionally, a pair of primers, if such pair of primers are not already provided as analyte-specific reagent(s) (ASR) being attached to or contained in said microparticles; OR
wherein said analyte of interest is a protein or other non-nucleic acid molecule, said detection reaction is an immunochemistry detection reaction, and said detection composition is a composition for performing such immunochemistry detection reaction and is provided in said kit in two separate compartments or containers; wherein said detection composition comprises, in a first compartment or container, necessary reagents for performing an immunochemistry detection reaction, such as a buffer, and a secondary antibody or secondary antibody fragment coupled to a suitable reporter enzyme and being specific for the same analyte as a primary antibody, antibody fragment, or non-antibody protein, used as analyte-specific reagent (ASR) in said immunochemistry reaction; and, optionally, a primary antibody, antibody fragment, or a non-antibody protein capable of specifically binding said protein analyte or other non-nucleic acid analyte, if such a primary antibody, antibody fragment, or non-antibody protein is not already provided as analyte-specific reagent(s) (ASR) being attached to or contained in said microparticles; and wherein said detection composition comprises, in a second compartment or container, as a detection reagent, a suitable substrate for said suitable reporter enzyme which substrate upon having been reacted by said reporter enzyme, becomes detectable, preferably optically detectable, more preferably detectable by fluorescence.

21. The kit according to any of claims 18 - 20, wherein each of said porous microparticles has the same analyte-specific reagent attached to its porous matrix or contains the same analyte-specific reagent.

22. The kit according to claim 21, wherein, in said plurality of porous microparticles, there are different subsets of microparticles,
with each subset
- having its distinct label component attached to, contained in or otherwise associated with said microparticles of said subset; and
all of said different subsets having
- the same analyte-specific reagent attached to or contained in said microparticles of said subsets, said analyte-specific reagent being specific for one analyte of interest;
such that said different subsets of microparticles are identical in terms of the analyte-specific reagent attached or contained, but differ by
- the respective label component attached to, contained in or otherwise associated with said microparticles of each subset;
with each subset being unambiguously defined and identifiable by said respective label component and being provided in a separate container.

23. The kit according to any of claims 21 - 22, wherein said kit is a kit for detecting one analyte of interest in a plurality of biological liquid samples, wherein the number of different subsets of microparticles provided in said kit equals the number of separate biological liquid samples provided.

24. The kit according to any of claims 18 - 20, wherein, in said plurality of porous microparticles, there are several different analyte-specific reagents attached to or contained in said microparticles.

25. The kit according to claim 24, wherein there are different subsets of microparticles,
with each subset
- having its distinct label component attached to, contained in or otherwise associated with said microparticles of said subset; and
wherein furthermore, in said plurality of porous microparticles, there are different classes of subsets of microparticles with each class of subsets
- having a different analyte-specific reagent attached to the porous matrix of said microparticles or contained in said microparticles; wherein, preferably, there are at least two different classes of subsets of microparticles, more preferably at least three or more different classes of subsets of microparticles;
such that said different subsets of microparticles differ by the respective label component attached to, contained in or otherwise associated with said microparticles of each subset; and each subset of microparticles forms part of one class of subsets of microparticles; with each subset being unambiguously defined and identifiable by the respective label component and the respective analyte-specific reagent and being provided in a separate container; and
such that said different classes of subsets of microparticles differ by the respective analyte-specific reagent attached to the porous matrix of said microparticles or contained in said microparticles; and each of said different classes comprises several subsets of microparticles, all of which subsets have the same analyte-specific reagent attached or contained.

26. The kit according to any of claims 24 - 25, wherein said kit is a kit for detecting more than one analyte of interest in a plurality of biological liquid samples, wherein, the number of different subsets of microparticles provided in said kit equals the number of separate biological liquid samples provided, multiplied by the number of analytes of interest to be detected, and wherein, in said kit, there are as many classes of subsets of microparticles provided as the number of analytes of interest to be detected.

27. A cartridge for performing a method of detecting and/or quantitating an analyte of interest in a plurality of biological liquid samples according to any of claims 1 - 17, wherein, preferably, said cartridge comprises a plurality of sample-specific modules, a plurality of storage chambers, at least one non-aqueous phase chamber for storing a non-aqueous phase, and either a single combined mixing and detection chamber, or a combination of a separate mixing chamber and a separate detection chamber;
wherein each sample-specific module comprises a sample compartment having its own separate sample inlet, each sample-specific module being configured to separately receive exactly one biological sample only, in the respective sample compartment; each sample-specific module being furthermore configured to receive microparticles in said sample compartment, said microparticles being as defined in any of claims 1 - 17; each sample-specific module being further configured to facilitate a phase-transfer of said microparticles from an aqueous environment to a non-aqueous environment.
